(19) Europäisches Patentamt
European Patent Office
Office européen des brevets

(11) Publication number: **0 027 744**
**B1**

(12) **EUROPEAN PATENT SPECIFICATION**

(45) Date of publication of patent specification: **14.03.84**

(21) Application number: **80303754.8**

(22) Date of filing: **23.10.80**

(51) Int. Cl.³: **C 07 D 333/20,**
C 07 D 409/12,
A 61 K 31/38, A 61 K 31/41
//(C07D409/12, 333/20,
249/14)

(54) **Thiophene derivatives, processes for the production thereof and pharmaceutical compositions containing them.**

(30) Priority: **23.10.79 GB 7936766
23.10.79 GB 7936762
29.02.80 GB 8006940
05.06.80 GB 8018404**

(43) Date of publication of application:
**29.04.81 Bulletin 81/17**

(45) Publication of the grant of the patent:
**14.03.84 Bulletin 84/11**

(84) Designated Contracting States:
**BE CH DE FR IT LI NL SE**

(56) References cited:
EP - A - 0 001 699
EP - A - 0 002 930
DE - A - 2 821 409

(73) Proprietor: **GLAXO GROUP LIMITED
Clarges House 6-12 Clarges Street
London W1Y 8DH (GB)**

(72) Inventor: **Judd, Duncan Bruce
61 Musley Lane
Ware Herts (GB)**
Inventor: **Bradshaw, John
22 Whiteley Close
Dane End, Ware Herts (GB)**
Inventor: **Clitherow, John Watson
54 Gilders
Sawbridgeworth Herts (GB)**
Inventor: **Price, Barry John
155 Ware Road
Hertford Herts (GB)**
Inventor: **MacKinnon, John Wilson Macfarlane
31 Trapstyle Road
Ware Herts (GB)**
Inventor: **Hayes, Roger
5 Sandringham Road
Potters Bar Herts (GB)**
Inventor: **Carey, Linda
15 Roan Walk
Royston Herts (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 027 744 B1**

Courier Press, Leamington Spa, England.

(74) Representative: **Marchant, James Ian et al,**
**Elkington and Fife High Holborn House**
**52/54 High Holborn**
**London WC1V 6SH (GB)**

# Thiophene derivatives, processes for the production thereof and pharmaceutical compositions containing them

This invention relates to novel heterocyclic derivatives having action on histamine receptors, to processes for the preparation thereof, to pharmaceutical compositions containing them and to their use in therapeutics.

Certain novel heterocyclic derivatives have now been found which have potent activity as $H_2$-antagonists. These compounds, which are more particularly described below, for example show inhibition of the secretion of gastric acid when this is stimulated via histamine receptors (Ash and Schild, Brit. J. Pharmacol, Chemother 1966, *27*, 427). Their ability to do so can be demonstrated in the perfused rat stomach using the method described in German Offenlegungsschrift No. 2,734,070, modified by the use of sodium pentobarbitone (50 mg/kg) as anaesthetic instead of urethane, and in conscious dogs equipped with Heidenhain pouches using the method described by Black *et al*, Nature 1972 *236* 385. Furthermore the compounds antagonise the effect of histamine on the contraction frequency of isolated guinea pig right atrium but do not modify histamine induced contractions of isolated gastro-intestinal smooth muscle which are mediated via $H_1$-receptors.

Compounds with histamine $H_2$-blocking activity may be used in the treatment of conditions where there is an advantage in lowering gastric acidity, particularly in gastric and peptic ulceration, as a prophylactic measure in surgical procedures, and in the treatment of allergic and inflammatory conditions where histamine is a known mediator. Thus they may be used for example, either alone, or in combination with other active ingredients in the treatment of allergic and inflammatory conditions of the skin.

The present invention provides compounds of the general formula (I)

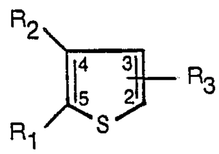

(I)

and physiologically acceptable salts and hydrates thereof, in which one of $R_1$ and $R_2$ represents hydrogen, halogen or a $C_{1-4}$ alkyl group which may be optionally substituted by hydroxy or $C_{1-4}$ alkoxy, and the other represents the group $R_4R_5NAlk$—

in which $R_4$ represents hydrogen, $C_{1-10}$ alkyl, $C_{3-8}$ cycloalkyl, $C_{3-6}$ alkenyl, $C_{3-6}$ alkynyl, $C_{1-6}$ trifluoroalkyl, $C_{1-6}$ alkyl substituted by phenyl, substituted phenyl, hydroxy, $C_{1-6}$ alkoxy, amino, $C_{1-6}$ alkylamino, di $C_{1-6}$ alkylamino or $C_{3-8}$ cycloalkyl,

or $R_4$ represents $C_{1-4}$ alkyl substituted by a 5 or 6 membered monocyclic heteroaryl ring containing one or more heteroatoms selected from oxygen, nitrogen and sulphur, which heteroaryl ring is unsubstituted or substituted by $C_{1-3}$ alkyl, $C_{1-3}$ alkoxy, hydroxy, amino-$C_{1-6}$ alkyl, $C_{1-6}$ alkylamino $C_{1-6}$ alkyl, di $C_{1-6}$ alkylamino $C_{1-6}$ alkyl or halogen, and which heteroaryl ring is linked to the alkyl group through either a carbon or a nitrogen atom,

and $R_5$ represents hydrogen or a $C_{1-4}$ alkyl group,

or $R_4$ and $R_5$ may, together with the nitrogen atom to which they are attached, form a 5 to 10 membered ring which may be saturated or may contain at least one double bond, may be unsubstituted or may be substituted by one or more $C_{1-3}$ alkyl groups e.g. methyl, or a hydroxy group and/or may contain another heteroatom selected from oxygen and sulphur;

Alk represents a straight or branched alkylene chain of 1 to 6 carbon atoms, preferably 1 to 4 carbon atoms;

$R_3$, which may be in either the 2- or 3-position, represents the group $—(CH_2)_nX(CH_2)_mNH—Z$ where X represents $—CH_2—$, —O— or —S—;

n represents zero, 1 or 2;

m represents 2, 3 or 4; and

Z represents either the group

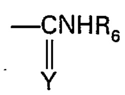

or the group

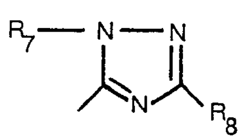

where Y represents S, O, $CHNO_2$ or $NR_9$ where $R_9$ is nitro, cyano, $C_{1-6}$ alkylsulphonyl, phenyl, sulphonyl or substituted phenyl sulphonyl;

$R_6$ represents hydrogen, $C_{1-6}$ alkyl, $C_{3-6}$ alkenyl, $C_{3-6}$ alkynyl, $C_{1-6}$ alkoxy $C_{1-6}$ alkyl or $C_{1-6}$ alkyl substituted by phenyl or substituted phenyl;

$R_7$ represents hydrogen, $C_{1-6}$ alkyl, $C_{3-6}$ alkenyl, $C_{1-6}$ alkyl substituted by phenyl or substituted phenyl, or $C_{2-6}$ alkyl substituted by hydroxy or $C_{1-6}$ alkoxy; and

$R_8$ represents hydrogen, $C_{1-6}$ alkyl, $C_{3-6}$ alkenyl, $C_{1-6}$ alkyl substituted by phenyl or substituted phenyl, hydroxy, $C_{1-6}$ alkyl, acyloxy $C_{1-6}$ alkyl where the acyl portion is benzoyl, substituted benzoyl, $C_{1-6}$ alkanoyl or $C_{2-7}$ alkanoyl substituted by phenyl or substituted phenyl, $C_{1-6}$ alkoxy $C_{1-6}$ alkyl, aryloxy $C_{1-6}$ alkyl in which the aryl group is phenyl or substituted phenyl, aryl $C_{1-6}$ alkyloxy $C_{1-6}$ alkyl in which the aryl group is phenyl or substituted phenyl, amino $C_{1-6}$ alkyl, $C_{1-6}$ alkylamino $C_{1-6}$ alkyl, di $C_{1-6}$ alkylamino $C_{1-6}$ alkyl, hydroxy or $C_{1-6}$ alkoxy, or the group $NR_{10}R_{11}$ where

$R_{10}$ represents hydrogen, $C_{1-6}$ alkyl, $C_{1-6}$ alkyl substituted by hydroxy or $C_{1-3}$ alkoxy, $C_{3-6}$ alkenyl, $C_{1-6}$ alkyl substituted by phenyl or substituted phenyl, or $C_{1-4}$ alkyl substituted by a 5 or 6 membered monocyclic heteroaryl ring containing one or more heteroatoms selected from oxygen, nitrogen and sulphur, which heteroaryl ring is unsubstituted or substituted by $C_{1-3}$ alkyl, $C_{1-3}$ alkoxy, hydroxy, amino $C_{1-6}$ alkyl, $C_{1-6}$ alkylamino $C_{1-6}$ alkyl, di $C_{1-6}$ alkylamino $C_{1-6}$ alkyl or halogen, and which heteroaryl ring is linked to the alkyl group through either a carbon or a nitrogen atom, and $R_{11}$ represents any of the groups defined for $R_{10}$ or may represent $COR_{12}$ where

$R_{12}$ represents hydrogen, $C_{1-6}$ alkyl, phenyl, substituted phenyl, $C_{1-6}$ alkyl substituted by phenyl or substituted phenyl, $C_{1-6}$ alkoxy, a 5 or 6 membered monocyclic heteroaryl ring containing one or more heteroatoms selected from oxygen, nitrogen and sulphur, which heteroaryl ring is unsubstituted or substituted by $C_{1-3}$ alkyl, $C_{1-3}$ alkoxy, hydroxy, amino $C_{1-6}$ alkyl, $C_{1-6}$ alkylamino $C_{1-6}$ alkyl, di $C_{1-6}$ alkylamino $C_{1-6}$ alkyl or halogen, or $C_{1-4}$ alkyl substituted by a 5 or 6 membered monocyclic heterocyclic ring containing one or more heteroatoms selected from oxygen, nitrogen and sulphur, which heteroaryl ring is unsubstituted or substituted by $C_{1-3}$ alkyl, $C_{1-3}$ alkoxy, hydroxy, amino $C_{1-6}$ alkyl, $C_{1-6}$ alkylamino $C_{1-6}$ alkyl, di $C_{1-6}$ alkylamino $C_{1-6}$ alkyl or halogen, and which heteroaryl ring is linked to the alkyl group through either a carbon or a nitrogen atom,

$R_{11}$ represents the group $SO_2R_{13}$ where $R_{13}$ represents $C_{1-6}$ alkyl, phenyl or substituted phenyl, or $R_{11}$ represents the group

$$\underset{\displaystyle E}{\overset{\displaystyle CNHR_{12}}{\|}}$$

where E represents oxygen or sulphur, and $R_{14}$ represents hydrogen, $C_{1-6}$ alkyl, $C_{3-8}$ cycloalkyl, phenyl, substituted phenyl or $C_{1-6}$ alkyl substituted by phenyl or substituted phenyl, or

$R_{10}$ and $R_{11}$ taken together represent the group $=CR_{15}R_{16}$ where $R_{15}$ represents phenyl, substituted phenyl or a 5 or 6 membered monocyclic heteroaryl ring containing one or more heteroatomes selected from oxygen, nitrogen and sulphur, which heteroaryl ring is unsubstituted or substituted by $C_{1-3}$ alkyl, $C_{1-3}$ alkoxy, hydroxy, amino $C_{1-6}$ alkyl, $C_{1-6}$ alkylamino $C_{1-6}$ alkyl, di $C_{1-6}$ alkylamino $C_{1-6}$ alkyl or halogen, and $R_{16}$ represents hydrogen or $C_{1-6}$ alkyl,

with the provisos that

where $R_2$ represents the group $R_4R_5NAlk$ then $R_3$ is in the 2- position; and

where $R_2$ represents hydrogen then $R_3$ is in the 3- position.

The term "alkyl" as a group or part of a group means that the group is straight or branched. Unless otherwise stated the group has, in particular, 1 to 4 carbon atoms, e.g. methyl or ethyl. The terms "substituted phenyl" and "substituted benzoyl" mean phenyl or benzoyl substituted in the phenyl ring with one or more $C_{1-3}$ alkyl or $C_{1-3}$ alkoxy groups or halogen atoms, e.g. fluorine.

Examples of acyloxy $C_{1-6}$ alkyl groups include acetoxymethyl, formyloxymethyl, benzoyloxymethyl and phenylacetoxymethyl.

Examples of heteroaryl rings within the definition of $R_4$ and $R_8$ are furyl, pyridyl, thiazolyl and thienyl.

The invention includes the compounds of formula (I) in the form of physiologically acceptable salts with inorganic and organic acids. Particularly useful salts include hydrochlorides, hydrobromides, sulphates, methanesulphonates, acetates, maleates, succinates, tartrates, citrates, benzoates and fumarates. The compounds of formula (I) and their salts may also form hydrates, which hydrates are also to be considered as part of the invention. The compounds of formula (I) can exhibit tautomerism and the formula is intended to cover all tautomers. Where optical isomers may exist the formula is intended to cover all diastereoisomers and optical enantiomers.

The compounds according to the invention preferably in the form of a salt, may be formulated for administration in any convenient way and the invention includes within its scope pharmaceutical compositions containing at least one compound according to the invention adapted for use in human or veterinary medicine. Such compositions may be formulated in a conventional manner using one or

more pharmaceutically acceptable carriers or excipients. Such compositions may also contain if required other active ingredients, e.g. $H_1$-antagonists.

Thus the compounds according to the invention may be formulated for oral, buccal, topical, parenteral or rectal administration. Oral administration is preferred.

For oral administration, the pharmaceutical composition may take the form of for example, tablets, capsules, powders, solutions, syrups or suspensions prepared by conventional means with acceptable excipients. For buccal administration the composition may take the form of tablets or lozenges formulated in conventional manner.

The compounds of the invention may be formulated for parenteral administration by bolus injection or continuous infusion. Formulations for injection may be presented in unit dosage form in ampoules, or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for reconstitution with a suitable vehicle, e.g. sterile pyrogen-free water before use.

The compounds of the invention may also be formulated in rectal compositions such as suppositories or retention enemas, e.g. containing conventional suppository bases such as cocoa butter or other glyceride.

For topical application, the compounds of the invention may be formulated as ointments, creams, gels, lotions, powders or sprays in a conventional manner.

For internal administration a convenient daily dosage regime of the compounds according to the invention would be 2 to 4 doses to the total of some 5 mg. to 2 g per day, preferably 5 to 500 mg. per day, dependent upon the condition of the patient.

Examples of suitable meanings for the groups $R_1$, $R_2$, $R_4$, $R_5$, $R_6$, $R_7$, $R_8$, Y and Alk are as follows:

$R_1$ or $R_2$ (when other than the group $R_4R_5NAlk$): a hydrogen atom, a bromine atom or a $C_{1-3}$ alkyl group (e.g. methyl, ethyl or isopropyl) or a $C_{1-3}$ alkoxy $C_{1-3}$ alkyl group (e.g. methoxymethyl) or a hydroxy $C_{1-3}$ alkyl group (e.g. hydroxymethyl);

or, when $R_1$ or $R_2$ is the group $R_4R_5NAlk$,

$R_4$: $C_{1-10}$ alkyl (e.g. methyl, ethyl, propyl, butyl, pentyl, hexyl, heptyl or decyl), $C_{5-7}$ cycloalkyl (e.g. cyclopentyl, cyclohexyl or cycloheptyl), $C_{3-6}$ alkenyl (e.g. allyl or 3,3-dimethylallyl), $C_{1-6}$ alkyl substituted by phenyl or substituted phenyl (e.g. phenyl $C_{1-6}$ alkyl such as benzyl or phenethyl), $C_{1-4}$ alkyl substituted by a trifluoromethyl group (e.g. 2,2,2,-trifluoroethyl), hydroxy $C_{2-4}$ alkyl (e.g. 3-hydroxypropyl), $C_{1-3}$ alkoxy $C_{2-4}$ alkyl (e.g. methoxyethyl or ethoxyethyl), di-$C_{1-3}$ alkylamino $C_{1-6}$ alkyl (e.g. dimethylaminoethyl, diethylaminoethyl or dimethylaminopropyl), or heteroaralkyl where the heterocyclic portion represents a furyl, thienyl, pyrrolyl, pyridinyl, pyrimidinyl, triazinyl, oxazolyl, triazolyl or thiazolyl ring and the alkylene portion is a methylene, ethylene or propylene grouping; and

$R_5$: hydrogen or a methyl or ethyl group; or

$R_4R_5N$ may represent a 5—8 membered ring optionally containing one double bond and/or substituted by one or two $C_{1-3}$ alkyl (e.g. methyl) groups or a hydroxy group and/or containing an oxygen or sulphur atom (e.g. pyrrolidino, piperidino, hexamethylenimino, heptamethylenimino, tetrahydropyridino, 4-hydroxypiperidino, 4—$C_{1-3}$ alkylpiperidino (e.g. 4-methylpiperidino), morpholino, 2,6-di-$C_{1-3}$ alkylmorpholino (e.g. 2,6-dimethylmorpholino), or thiamorpholino);

$R_6$: hydrogen, $C_{1-4}$ alkyl (e.g. methyl or ethyl) or $C_{1-3}$ alkoxy $C_{2-4}$ alkyl (e.g. methoxyethyl);

Y: $CHNO_2$ or $NR_9$ where $R_9$ is nitro, cyano, $C_{1-4}$ alkylsulphonyl (e.g. methylsulphonyl), phenylsulphonyl or substituted phenylsulphonyl;

$R_7$: hydrogen, $C_{1-4}$ alkyl (e.g. methyl, ethyl or propyl), or hydroxy-$C_{2-4}$ alkyl (e.g. 2-hydroxyethyl);

$R_8$: hydrogen, hydroxy, $C_{1-4}$ alkyl (e.g. methyl, ethyl or isobutyl), hydroxy $C_{1-4}$ alkyl (e.g. hydroxymethyl, 2-hydroxyethyl or 1-hydroxy-1-methylethyl), $C_{1-3}$ alkoxy $C_{1-4}$ alkyl (e.g. methoxymethyl or methoxyethyl), phenyl $C_{1-3}$ alkyl (e.g. benzyl or phenethyl), $C_{2-4}$ alkanoyloxy $C_{1-4}$ alkyl (e.g. acetoxymethyl), amino $C_{1-4}$ alkyl (e.g. aminomethyl), amino, $C_{1-4}$ alkylamino (e.g. methylamino or ethylamino) or di-$C_{1-4}$ alkylamino (e.g. dimethylamino, diethylamino or dipropylamino), phenyl $C_{1-3}$ alkylamino (e.g. benzylamino), or a heteroaryl $C_{1-3}$ alkylamino group where the heteroaryl ring is 5 or 6 membered and contains one heteroatom (e.g. 3- or 4- pyridylmethyl); or the group $NHCOR_{12}$ where $R_{12}$ represents hydrogen, $C_{1-3}$ alkyl (e.g. methyl or ethyl), $C_{1-3}$ alkoxy (e.g. methoxy or ethoxy), furyl, pyridyl, thiazolyl, thienyl, or phenyl optionally substituted by a $C_{1-3}$ alkyl (e.g. methyl) or $C_{1-3}$ alkoxy (e.g. methoxy) group; or the group $NHSO_2R_{13}$ where $R_{13}$ represents $C_{1-3}$ alkyl (e.g. methyl), or phenyl optionally substituted by a $C_{1-3}$ alkyl (e.g. methyl) or $C_{1-3}$ alkoxy (e.g. methoxy) group; or the group $NHCONHR_{14}$ where $R_{14}$ is $C_{1-3}$ alkyl (e.g. methyl), $C_{5-7}$ cycloalkyl (e.g. cyclohexyl), or phenyl optionally substituted by a $C_{1-3}$ alkyl (e.g. methyl) or $C_{1-3}$ alkoxy (e.g. methoxy) group; or the group $N{=}CHR_{15}$ where $R_{15}$ is a phenyl or pyridyl (e.g. 3- or 4- pyridyl) group. The group Alk may be for example the group $(CH_2)_p$ where p is 1, 2 or 3.

In particular the groups $R_1$, $R_2$, $R_4$, $R_5$, $R_6$, $R_7$, $R_8$ and Y may have the following meanings:

$R_1$ or $R_2$ (when other than the group $R_4R_5NAlk$): hydrogen or $C_{1-4}$ alkyl (e.g. methyl);

$R_4$: $C_{1-7}$ alkyl (e.g. methyl, propyl, butyl, isobutyl or heptyl), $C_{1-4}$ alkyl substituted by a trifluoromethyl group (e.g. 2,2,2-trifluoroethyl), $C_{2-4}$ alkyl substituted by a hydroxy or di $C_{1-3}$ alkylamino group (e.g. 3-hydroxypropyl or dimethylaminoethyl, $C_{5-7}$ cycloalkyl (e.g. cyclohexyl), alkenyl (e.g. allyl), phenyl

$C_{1-3}$ alkyl (e.g. benzyl), or a heteroaryl $C_{1-3}$ alkyl group where the heteroaryl ring is 5 or 6 membered and contains one heteroatom (e.g. furylmethyl);

$R_5$: hydrogen or methyl; or

$R_4R_5N$ may represent a 5 to 7 membered ring, optionally containing a double bond or an alkyl (e.g. methyl) group (e.g. piperidino, 4-methylpiperidino, pyrrolidino, hexamethylenimino or tetrahydropyridino);

$R_6$: hydrogen or $C_{1-3}$ alkyl (e.g. methyl)

Y: $CHNO_2$ or $NR_9$ where $R_9$ is nitro or methylsulphonyl;

$R_7$: hydrogen, methyl, ethyl or 2-hydroxyethyl;

$R_8$: hydroxy, phenyl $C_{1-3}$ alkyl (e.g. benzyl), $C_{1-4}$ alkyl substituted by hydroxy, $C_{1-3}$ alkoxy, $C_{2-4}$ alkanoyloxy or amino (e.g. hydroxymethyl, 2-hydroxyethyl, acetoxymethyl or aminomethyl); amino, di-$C_{1-4}$ alkylamino (e.g. diethylamino); $NHCOR_{12}$ where $R_{12}$ represents hydrogen, $C_{1-3}$ alkyl (e.g. methyl), $C_{1-3}$ alkoxy (e.g. ethoxy), or phenyl; $NHCONHR_{14}$ where $R_{14}$ represents phenyl; or $N{=}CHR_{15}$ where $R_{15}$ is phenyl or pyridyl (e.g. 4-pyridyl). Alk is particularly a methylene or ethylene group, more particularly methylene.

When $R_3$ is in the 3-position preferably $R_2$ represents hydrogen.

When $R_3$ is in the 2-position preferably $R_2$ represents alkyl (e.g. methyl).

When X is sulphur, n is preferably 1 and m is preferably 2.

Three preferred groups of compounds are as follows:

(i) compounds in which $R_3$ is in the 3-position and $R_1$ is the group $R_4R_5NAlk$, and $R_2$ is more particularly hydrogen;

(ii) compounds in which $R_3$ is in the 2-position and $R_2$ is the group $R_4R_5NAlk$;

(iii) compounds in which $R_3$ is in the 2-position and $R_1$ is the group $R_4R_5NAlk$, and $R_2$ is more particularly alkyl (e.g. methyl).

A preferred group of compounds of formula (I) where Z represents the group

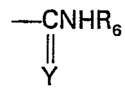

are those in which Y represents the group $CHNO_2$ and $R_6$ is more particularly alkyl (e.g. methyl).

A preferred group of compounds of formula (I) where Z represents the group

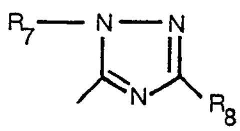

are those in which $R_7$ is hydrogen or methyl, and $R_8$ is amino, hydroxy $C_{1-4}$ alkyl, $C_{1-3}$ alkoxy $C_{1-4}$ alkyl, benzyl, formamido, $C_{2-4}$ alkanoylamino, amino $C_{1-4}$ alkyl, $C_{2-4}$ alkanoyloxy $C_{1-4}$ alkyl, hydroxy, benzoylamino or phenylcarbamoylamino.

A particularly preferred group of compounds of formula (I) are those of formula (II)

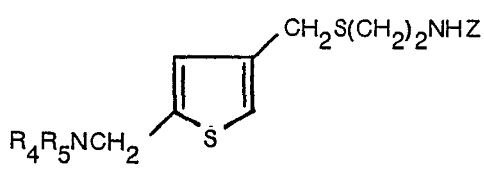

(II)

where $R_4$ and $R_5$ are methyl groups or together with the nitrogen atom to which they are attached form a pyrrolidino, piperidino or hexamethyleneimino group, and Z represents either

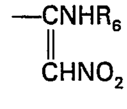

where $R_6$ is methyl, or the group

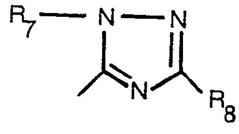

where $R_7$ is hydrogen or methyl, and $R_8$ is amino, hydroxy $C_{1-4}$ alkyl, $C_{1-3}$ alkoxy $C_{1-4}$ alkyl, benzyl, formamido, $C_{2-4}$ alkanoylamino, amino $C_{1-4}$ alkyl, $C_{2-4}$ alkanoyloxy $C_{1-4}$ alkyl, hydroxy, benzoylamino or phenylcarbamoylamino.

The following compounds and their physiologically acceptable salts are particularly preferred:

N-methyl-N′-[2-[[5-(dimethylaminomethyl)-3-thienylmethyl]thio]ethyl]-2-nitro-1,1-ethenediamine

N-methyl-N′-[2-[[5-(1-pyrrolidinylmethyl-3-thienylmethyl]-thio]ethyl]-2-nitro-1,1-ethenediamine

N-methyl-N′-[2-[[5-(1-piperidinylmethyl)-3-thienylmethyl]thio]ethyl]-2-nitro-1,1-ethenediamine

N-[2-[[5-(dimethylaminomethyl)-4-methyl-2-thienylmethyl]thio]ethyl-N′-methyl-2-nitro-1,1-ethenediamine

N-[3-[5-(dimethylaminomethyl)-3-thienylmethoxy]propyl]-N′-methyl-2-nitro-1,1-ethenediamine

1-methyl-$N^5$-[2-[[5-[(dimethylamino)methyl]-4-methyl-2-thienylmethyl]thio]ethyl-1H-1,2,4-triazole-3,5-diamine

1-methyl-5-[[2-[[5-(dimethylaminomethyl)-3-thienylmethyl]-thio]ethyl]amino]-1H-1,2,4-triazole-3-methanol

5-[[2-[[[5-(dimethylaminomethyl)-4-methyl]-2-thienylmethyl]thio]ethyl]amino]-1-methyl-1H-1,2,4-triazole-3-methanol

5-[[2-[[5-(dimethylaminomethyl)-3-thienyl]methyl]thio]ethyl]amino-3-phenylmethyl-1H-1,2,4-triazole.

According to one aspect the invention provides compounds of formula (I) in which one of $R_1$ and $R_2$ represents hydrogen and the other represents $R_4R_5NAlk$ and $R_3$ represents

$$—(CH_2)_nX(CH_2)_mNH\underset{\underset{Y}{\|}}{C}NHR_6$$

except that Y does not represent $NR_9$ where $R_9$ is alkylsulphonyl or arylsulphonyl.

According to another aspect the invention provides compounds of formula (I) in which one of $R_1$ and $R_2$ represents hydrogen and the other represents $R_4R_5NAlk$ and $R_3$ represents

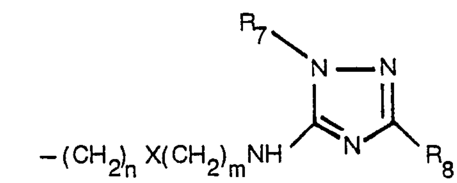

where $R_8$ represents hydrogen, $C_{1-6}$ alkyl, $C_{3-6}$ alkenyl, $C_{1-6}$ alkyl substituted by phenyl or substituted phenyl, hydroxy $C_{1-6}$ alkyl, $C_{1-6}$ alkoxy $C_{1-6}$ alkyl, hydroxy or $C_{1-6}$ alkoxy or $R_8$ represents the group $NR_{10}R_{11}$ where

$R_{10}$ represents hydrogen, $C_{1-6}$ alkyl, hydroxy $C_{1-6}$ alkyl, $C_{3-6}$ alkenyl or $C_{1-6}$ alkyl substituted by phenyul or substituted phenyl and $R_{11}$ represents hydrogen, $C_{1-6}$ alkyl, hydroxy $C_{1-6}$ alkyl, or the group $COR_{12}$ where

$R_{12}$ represents hydrogen, $C_{1-6}$ alkyl, phenyl substituted phenyl, $C_{1-6}$ alkoxy, or $C_{1-6}$ alkyl substituted by phenyl or substituted phenyl, or

$R_{11}$ represents the group $SO_2R_{13}$ where $R_{13}$ represents $C_{1-6}$ alkyl, phenyl or substituted phenyl

$R_{10}$ and $R_{11}$ taken together represent the group $=CR_{15}R_{16}$ where $R_{15}$ represents phenyl or substituted phenyl and $R_{16}$ represents hydrogen or $C_{1-6}$ alkyl.

According to a further aspect the invention provides compounds of formula (I) in which $R_1$ is $R_4R_5NAlk$—, $R_2$ is halogen or a $C_{1-4}$ alkyl group which may be optionally substituted by hydroxy or $C_{1-4}$ alkoxy and $R_3$, which is in the 2- position represents

$$—(CH_2)_nX(CH_2)_mNH\underset{\underset{Y}{\|}}{C}NHR_6$$

except that $R_4$ and $R_5$ do not represent alkynyl or heteroaralkyl and $R_4R_5N$ does not represent a 9 or 10 membered ring.

According to a still further aspect the invention provides compounds of formula (I) in which $R_1$ is $R_4R_5NAlk$—, $R_2$ is halogen or a $C_{1-4}$ alkyl group which may be substituted by hydroxy or $C_{1-4}$ alkoxy and $R_3$, which is in the 2- position represents

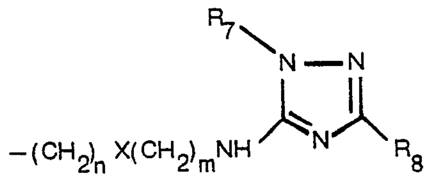

except that $R_4$ and $R_5$ do not represent alkynyl or heteroaralkyl and $R_4R_5N$ does not represent a 9 or 10 membered ring.

It will be appreciated in the methods for the preparation of the compounds of formula (I) given below, that for certain reaction steps it may be necessary to protect various reactive substituents in the starting materials for a particular reaction and subsequently to remove the protecting group. Such protection and subsequent deprotection may be particularly pertinent when $R_4$ and $R_5$ and/or $R_6$ are hydrogen, and/or when $R_7$ is an alkyl group bearing a hydroxy substituent and/or $R_8$ isn an alkyl group bearing a hydroxy or a primary or secondary amino substituent. Standard protection and deprotection procedures can be employed, for example formation of phthalimide, with subsequent cleavage by treatment with a hydrazine, e.g. hydrazine hydrate or a primary amine for example methylamine.

In describing the processes which may be used for preparing the compounds for formula (I) or intermediates useful in the preparation thereof, any of $R_1$ to $R_{16}$, Alk, X, Y, Z, E, n and m are as defined in formula (I) unless otherwise stated.

Compounds of formula (I) where Z represents the group

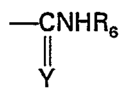

may be made by reacting an amine of the formula $R_{17}NH_2$ (III) with a compound of general formula

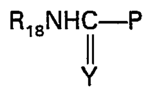

(IV)

wherein one of the groups $R_{17}$ and $R_{18}$ represents the group

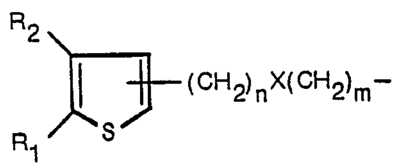

and the other represents the group $R_6$, and P is a leaving group such as halogen, alkylthio (preferably methylthio) or alkoxy.

Compounds of formula (IV) may be prepared by reacting the amine (III) with a compound of formula (V)

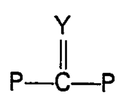

(V)

where P is as defined in formula (IV).

The above reactions may be effected in the absence or presence of a solvent e.g. ethanol, dioxan, acetonitrile, water or aqueous ethanol at a temperature from ambient to reflux. In the absence of a solvent, the reaction may be carried out by heating a mixture of the reactants at for example 100—120°C.

Compounds of formula (I) in which Z represents the group

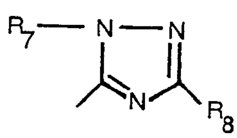

where $R_8$ is other than an alkoxy or acyloxyalkyl group or the group $—N{=}CR_{15}R_{16}$ may be prepared by cyclisation of a compound of formula (VI)

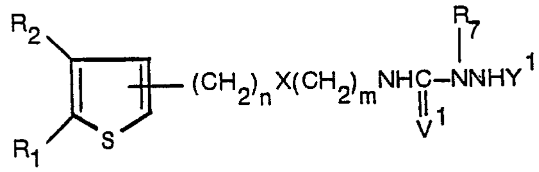

(VI)

in which $V^1$ is

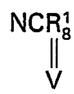

or NCN and $Y^1$ is hydrogen where V is oxygen or sulphur and $R_8^1$ is a group as defined in formula (I) for $R_8$ or a group convertible thereto under the conditions of the cyclisation reaction or represents halogen or alkoxy; or $V^1$ is NH and $Y^1$ is

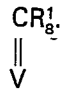

In a particular embodiment of the above process compounds of formula (I) in which the group Z represents

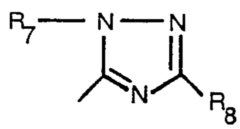

where $R_8$ is amino, hydroxy or a carbon-linked group may conveniently be prepared by cyclisation of a compound of formula (VII)

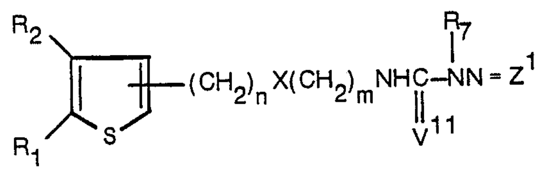

(VII)

where $V^{11}$ represents the group NCN or

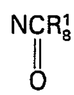

$Z^1$ represents two hydrogen atoms. The reaction may be carried out in the absence or presence of a suitable solvent, for example, toluene, ethanol, methanol, isopropanol, acetonitrile or water, at a temperature from room temperature to reflux.

It may be convenient to prepare *in situ* compounds of formula (VII) in which $Z^1$ represents two hydrogen atoms by treating a compound of formula (VII) where $Z^1$ represents a divalent protecting group which can readily be removed to yield two hydrogen atoms, for example a benzylidene group, with an acid, e.g. hydrochloric acid, preferably with heating and under such conditions cyclisation to give compounds of formula (I) will normally occur.

When preparing compounds of formula (I) in which $R_8$ is a hydroxyalkyl group it is preferable that in the intermediate (VII) the hydroxy group is in protected form e.g. as an acyloxy derivative such as an alkanoyloxy or aroyloxy derivative. The protecting group will normally be removed during the cyclisation process.

Compounds of formula (I) where Alk represents $CH_2$ and where Z represents the group

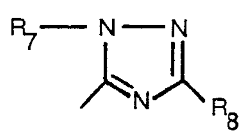

in which $R_8$ is other than the group $—N{=}CR_{15}R_{16}$ may be prepared by reducing the intermediate formed from reacting a compound of formula (VIII)

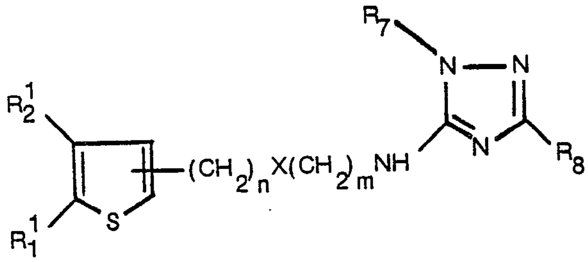

(VIII)

in which one of $R_1^1$ and $R_2^1$ represents hydrogen, halogen or a $C_{1-4}$ alkyl group which may be optionally substituted by hydroxy or $C_{1-4}$ alkoxy and the other represents the group —CHO with a reagent $R_4R_5NH$. Reaction may be effected by treatment with ammonia or an amine $R_4R_5NH$ in a solvent e.g. tetrahydrofuran or an alkanol such as ethanol or methanol, followed by reduction e.g. with a hydride reducing agent such as an alkali metal or alkaline earth metal borohydride, e.g. sodium borohydride, or aluminium hydride or lithium aluminium hydride or with hydrogen and a metal catalyst e.g. palladium or platinum.

Alternatively, for the production of compounds of formula (I) in which $R_8$ is other than $—N{=}CR_{15}R_{16}$, a compound of formula (I) in which $R_4$ is hydrogen may be reacted with an aldehyde $R_4^1$—CHO under the reducing conditions described above, the group $R_4^1$ having a meaning such that the resulting group $R_4^1CH_2$ represents a group $R_4$ as defined above. This method applies particularly to the preparation of compounds of formula (I) in which $R_4$ is heteroaralkyl or an optionally substituted alkyl chain containing up to 10 carbon atoms.

In certain instances compounds of formula (I) where Z represents the group

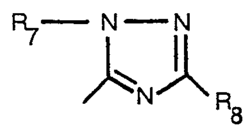

may be prepared by methods involving interconversion of the substituent $R_8$.

Thus compounds in which $R_8$ is an acyloxyalkyl group may be prepared by treating the corresponding hydroxyalkyl compound with an appropriate acid, e.g. acetic or benzoic acid at elevated temperatures e.g. 80—120°C in the absence or presence of a solvent such as toluene.

Compounds of formula (I) in which $R_8$ represents the group $—N{=}CR_{15}R_{16}$ may be prepared from compounds of formula (I) in which $R_8$ is an $NH_2$ group by reaction with an aldehyde or ketone $R_{15}R_{16}CO$ in a solvent such as benzene, ethanol, or methanol. The reaction is preferably carried out with heating, e.g. at reflux.

Compounds of formula (I) in which $R_8$ is the group $NR_{10}R_{11}$ where $R_{11}$ is $—COR_{12}$, $—SO_2R_{13}$ or $—C({=}E)NHR_{14}$ may be prepared by treating a compound of formula (I) in which $R_8$ is the group $—NHR_{10}$ and $R_1$, $R_2$, $R_7$ and $R_{10}$ are as defined in Formula (I) or are groups readily convertible thereto with a reagent capable of replacing the hydrogen atom in the group $NHR_{10}$ by the group $R_{11}$ where $R_{11}$ is as defined above.

Thus, for example, reaction with an activated derivative of either a carboxylic acid $R_{12}COOH$ or a sulphonic acid $R_{13}SO_3H$ or reaction with an isocyanate or isothiocyanate $R_{14}NCE$ in which $R_{14}$ has any of the meanings defined for $R_{14}$ in formula (I) except hydrogen or represents an alkali metal atom such as potassium or sodium or an alkoxycarbonyl group, e.g. ethoxycarbonyl, gives a compound of formula (I) in which $R_{11}$ is respectively the group $COR_{12}$, $SO_2R_{13}$ or $C({=}E)NHR_{14}$.

Suitable activated derivatives include acid halides e.g. acid chlorides, alkylchloroformates, acid anhydrides including mixed anhydrides (e.g. acetic formic anhydride), or esters such as alkyl esters, orthoesters and (1-alkyl-2-pyridinyl) esters.

The reaction with an acid halide is preferably carried out in the presence of a base e.g. an inorganic base such as sodium hydroxide or an organic base such as triethylamine or pyridine. The reaction with an alkylchloroformate is preferably carried out in the presence of a base, e.g. potassium carbonate or triethylamine, in a solvent such as dimethylformamide. The reaction with an acid anhydride may be carried out in the absence or presence of solvent such as pyridine.

In the reaction with an isocyanate or isothiocyanate compounds of formula (I) in which $R_{14}$ is other than hydrogen are conveniently prepared by carrying out the reaction in a solvent such as acetonitrile at an elevated temperature, e.g. reflux. Compounds of formula (I) in which $R_{14}$ is hydrogen may be prepared by heating with an appropriate organic isocyanate or isothiocyanate such as ethylcarbonisothiocyanatidate, at an elevated temperature followed by hydrolysis of the resulting ester, for example with a base such as aqueous ethanolic sodium hydroxide.

Compounds of formula (I) may also be prepared by treating a compound of formula (I) in which the group $R_4R_5N$ is replaced by a quaternary ammonium group (e.g. a trimethylammonium group) with an amine $R_4R_5NH$. The reaction may be carried out by heating the quaternary ammonium compound in the amine (e.g. at 100 to 120°C) or the reaction may be carried out in the presence of a solvent such as an alkanol (e.g. ethanol) at reflux. This process is particularly applicable to compounds in which Alk is $CH_2$.

Amines of formula (III) may be made by a number of methods depending on the precise structure of the compound.

Thus amines of formula (IX)

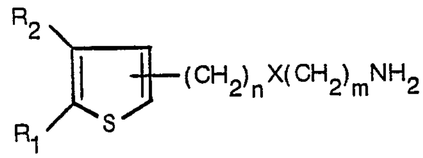

(IX)

in which n is 1 or 2 and X is oxygen or sulphur may be prepared from the corresponding alcohol of formula (X)

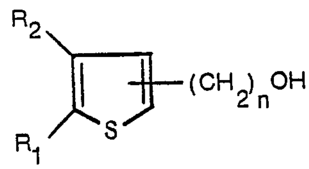

(X)

in which n is 1 or 2.

For example amines of formula (IX) in which n is 1 and X is sulphur may be prepared by reacting an alcohol of formula (X) in which n is 1 with an appropriate aminoalkane thiol salt (e.g. crystamine hydrochloride) in a concentrated mineral acid (e.g. hydrochloric acid).

Amines of formula (IX) in which n is 2 and X is sulphur may be prepared by converting an alcohol of formula (X) in which n is 2 into the corresponding haloalkyl compound using for example thionyl chloride or phosphorus tribromide, or mesylate by reaction with methanesulphonylchloride, followed by treatment with an appropriate aminoalkylthiol (e.g. cysteamine) in a solvent such as ethanol and in the presence of a base (e.g. sodium ethoxide).

Amines of formula (IX) in which n is 1 or 2 and X is oxygen may be prepared by treating an alcohol of formula (X) in which n is 1 or 2 with a suitable base (e.g. potassium t-butoxide) in a solvent (e.g. dimethylformamide) followed by the addition of an appropriate haloalkylamine (e.g. chloropropylamine).

The alcohols of formula (X), as well as the intermediates (XXII) and (XXIV) below, may be made by a variety of processes based on conventional methods in thiophene chemistry (Advances in Heterocyclic Chemistry Volume 1, 1963, page 2—116, Ed. A.R. Katritzky, Academic Press, London and New York; and Comprehensive Chemistry Volume 4, page 787, Ed. P.G. Sammes, Pergamon Press, Oxford). Some representative routes and reagents are given in each case.

Alcohols of formula (X) in which $R_1$ is the gorup $R_4R_5NCH_2$, n is 1 and the hydroxymethyl substituent is in either the 2- or 3-position may be prepared by treating a compound of formula (XI)

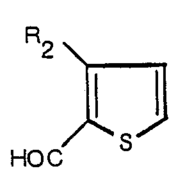

(XI)

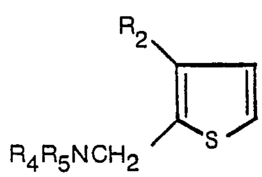

(XII)

with an amine $R_4R_5NH$ under reducing conditions as described previously for the preparation of compounds of the invention, to give a thiophenemethanamine of formula (XII) which may be subsequently reacted with paraformaldehyde in a concentrated mineral acid (e.g. hydrochloric acid) and acetic acid to introduce the hydroxymethyl group at either the 2- or 3- position.

Alcohols of formula (X) in which $R_2$ is the group $R_4R_5NCH_2$, n is 1 and the hydroxymethyl substituent is at the 2-position may be prepared by reacting a compound of formula (XIII)

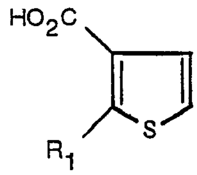

(XIII)

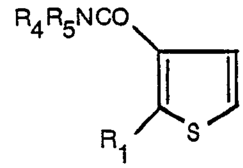

(XIV)

with for example oxalyl chloride in a solvent (e.g. benzene) and preferably in the presence of a catalyst (e.g. pyridine) followed by treatment with an amine $R_4R_5NH$ to give an amide of formula (XIV) which is subsequently reduced with a complex metal hydride e.g. lithium aluminium hydride in a solvent such as tetrahydrofuran to give a compound of formula (XV).

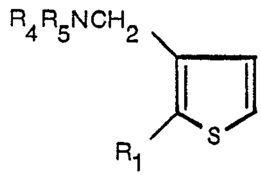

(XV)

The 2-hydroxymethyl group is then introduced by reacting the compound of formula (XV) with formaldehyde or a precursor of formaldehyde such as paraformaldehyde, in a concentrated mineral acid, e.g. hydrochloric acid or acetic acid.

Alcohols of formula (X) in which $R_1$ is the group $R_4R_5NCH_2$, $R_2$ is hydrogen, n is 1 and the hydroxymethyl substituent is in the 3-position may be prepared from a thiophene 3-carboxylate of formula (XVI)

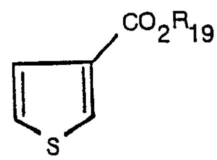

(XVI)

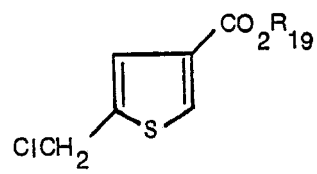

(XVII)

where $R_{19}$ is an alkyl group. This compound is halomethylated e.g. chloromethylated using for example formaldehyde, or a precursor of formaldehyde such as paraformaldehyde, and gaseous hydrogen chloride in a solvent such as chloroform and in the presence of zinc chloride to give a compound of formula (XVII). The amino group $R_4R_5N—$ is then introduced into the compound of formula (XVII) by reaction with an amine $R_4R_5NH$, in a suitable solvent such as ether, to give a compound of formula (XVIII)

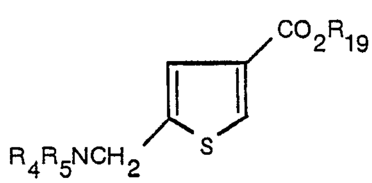

(XVIII)

Introduction of the 3-hydroxymethyl group is effected by reduction of the 3-carboxylate using for example lithium aluminium hydride in a suitable solvent such as ether.

Alcohols of formula (X) in which n is 2 may be prepared by lithiating a halothiophene of formula (XIX)

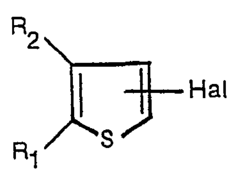

(XIX)

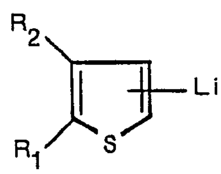

(XX)

(where Hal is halogen e.g. bromine) using n-butyl lithium in a suitable solvent (e.g. tetrahdyrofuran) at a low temperature (e.g. −78°), followed by treatment of the resulting lithio derivative (XX) with ethylene oxide in a solvent such as tetrahydrofuran, to give the desired hydroxymethyl compound (X, n=2).

In a modification of this process a halothiophene of formula (XIX) in which $R_1$ is a group convertible to $R_4R_5NCH_2—$ (e.g. an aldehyde grouping protected as an acetal) and the group Hal is at

the 3-position may be lithiated and treated with ethylene oxide as described above to give a hydroxyethyl compound of formula (XXI).

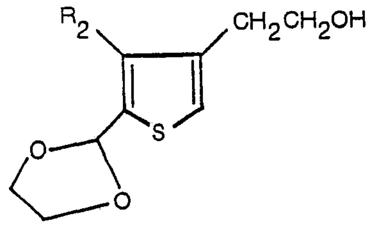

(XXI)

Subsequent treatment with an amine $R_4R_5NH$ under reducing conditions as described previously for preparing compounds of the invention affords an alcohol of formula (X) in which $R_1$ is the group $R_4R_5NCH_2$, n is 2 and the hydroxyethyl substituent is at the 3-position.

Amines of formula (IX) in which X is $CH_2$ may be prepared from an appropriate haloalkylthiophene of formula (XXII)

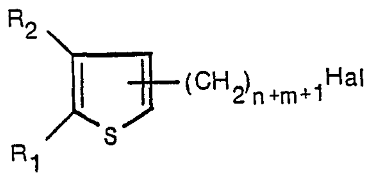

(XXII)

(in which Hal is halogen) by treatment with for example potassium phthalimide in a solvent (e.g. dimethylformamide), followed by deprotection of the phthalimido derivative using for example hydrazine hydrate.

The haloalkylthiophenes of formula (XXII) may be prepared for example by treating a lithio derivative of formula (XX) with an $\alpha,\omega$-dihaloalkane (e.g. $Br(CH_2)_n+_m+_1{}^{Br}$) in a solvent such as tetrahydrofuran to give an intermediate of formula (XXII).

Amines of formula (IX) in which X is $CH_2$ may also be prepared by methods involving conventional homologation of an alkyl chain. Thus for example a haloalkyl (e.g. chloroethyl) thiophene of formula (XXIII)

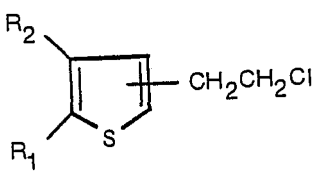

(XXIII)

may be treated with for example sodium cyanide in dioxan to give the corresponding cyanoethyl compound, which may then be reduced (using for example lithium aluminium hydride in tetrahydrofuran) to give the corresponding aminopropyl compound.

Alternatively a chloroethyl compound of formula (XXIII) may be treated with malonic ester to give, after decarboxylation, the corresponding butyric acid derivative. This may be treated with thionyl chloride for example, followed by treatment with ammonia, to give the corresponding amide which may then be reduced using for example lithium aluminium hydride to give the corresponding aminobutyl compound.

Amines of formula (IX) in which n is zero and X is oxygen may be prepared from an appropriate alkoxythiophene (XXIV)

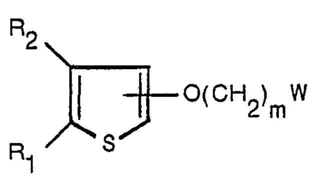

(XXIV)

where W is a halogen atom (e.g. chlorine or bromine) or a leaving group (e.g. mesylate), by a similar method to that described above for the preparation of amines of formula (IX) in which X is $CH_2$ from the corresponding haloalkyl thiophene.

Alkoxythiophenes of formula (XXIV) may be prepared for example by treating a halo (e.g. bromo)

thiophene of formula (XIX) with a diol $HO(CH_2)_mOH$ in the presence of a base (e.g. sodium hydride) and cuprous oxide, and preferably with the addition of potassium iodide, to give a hydroxyalkoxy compound of formula (XXV)

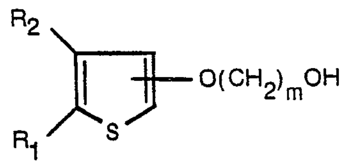

(XXV)

which may be treated with for example, thionyl chloride, phosphorus tribromide or methanesulphonyl chloride to give the alkoxythiophene of formula (XXIV).

Amines of formula (IX) in which n is zero and X is sulphur may be prepared by treating a lithio derivative of formula (XX) with elemental sulphur, followed by reaction with an appropriate haloalkylamine (e.g. chloropropylamine) preferably in protected form, e.g. as a phthalimide with subsequent cleavage of the phthalimide group using for example hydrazine hydrate.

Compounds of formula (I) and amines of formula (IX) in which Alk is an alkylene group other than $CH_2$ may be prepared from the intermediates described above in which Alk is $CH_2$ or a group convertible thereto (e.g. an ester group) by conventional means for ascending a homologous series. Thus for example a hydroxymethyl group may be converted into a chloromethyl group, which may then be converted into an aminoethyl or aminopropyl group by methods analogous to those described for amines of formula (IX) in which X is $CH_2$, and thence into the group $R_4R_5NAlk$ where Alk contains two or three methylene groups.

Intermediates of formulae (VI) and (VII) may be prepared from amines of formula (IX) by methods analogous to described in British Patent Specification No. $2023133_A$ and European Patent Specification Publication No. 0016565.

Intermediates of formula (VIII) in which the aldehyde grouping is preferably in protected form (e.g. as an acetal) may be prepared by the methods described above for preparing compounds of the invention except that in the appropriate intermediates the group $R_4R_5NAlk$ is replaced by a protected aldehyde group (e.g. an acetal grouping).

Where the product of any of the above processes is a free base and a salt is required, the salt may be formed in a conventional manner. Thus, for example, a generally convenient method of forming the salts is to mix appropriate quantities of the free base and the acid in an appropriate solvent(s), e.g. an alcohol such as ethanol or an ester such as ethyl acetate.

The invention is illustrated by, but not limited to, the following Examples and Preparations. In the exemplification temperatures are in °C and t.l.c. refers to thin layer chromatography carried out on silica using one of the following solvent systems unless otherwise stated.

System A: Methanol: 0.88 ammonia (79:1)
System B: Ethyl acetate isopropanol:water: 0.88 ammonia (25:15:8:2)

Preparation 1

(a) *Methyl 5-(dimethylaminomethyl)-3-thiophene carboxylate*

A solution of methyl 5-(chloromethyl)-3-thiophene-carboxylate (1.9 g) in dry diethylether (100 ml) was treated with anhydrous dimethylamine (5 ml). After 6 hours the solvent was removed *in vacuo* and the residue was dissolved in 5M hydrochloric acid (20 ml). The aqueous solution was washed with diethylether, basified with 5M sodium hydroxide (30 ml) and extracted with ether. The ethereal extracts were evaporated to give an oily residue which was distilled to yield the *title compound* as a colourless oil (1.6 g) b.p. 120—130°/0.5 mm.

The picrate salt was formed in and recrystallised from ethanol. m.p. 165°.

The following compounds were similarly prepared from methyl-5-(chloromethyl)-3-thiophene-carboxylate (A) and the corresponding amines:—

(b) A (7 g) and pyrrolidine (10 ml) gave methyl 5-(1-pyrrolidinylmetyl)-3-thiophene carboxylate (3.3 g), Picrate sale m.p. 122—3°.

(c) A (6 g) and piperidine (10 ml) gave methyl 5-(1-piperidinylmethyl)-3-thiophenecarboxylate (3.6 g), Picrate salt m.p. 186°.

Preparation 2

(a) *5-(Dimethylaminomethyl)-3-thiophenemethanol*

A solution of methyl 5-(dimethylaminomethyl)-3-thiophenecarboxylate (1.5 g) in diethylether (50 ml) was treated with lithium aluminium hydride (0.21 g). After 1 hour water (2 ml) was added and the solution was filtered through diatomaceous earth. Evaporation of the filtrate gave an oily residue which distilled to give the *title compound* as an oil (1.2 g) b.p. (120°/0.1 mm).

The oxalate salt was formed in and recrystallised from ethanol m.p. 107—8°.

The following compounds were similarly prepared from the corresponding ester.

(b) Methyl 5-(1-pyrrolidinylmethyl)-3-thiophenecarboxylate (3.2 g) gave 5-(1-pyrrolidinylmethyl-3-thiophenemethanol (2.4 g) Oxalate salt m.p. 111—2°.

(c) Methyl 5-(1-piperidinylmethyl)-3-thiophenecarboxylate (3.6 g) gave 5-(1-piperidinylmethyl)-3-thiophenemethanol (2.9 g). Oxalate salt m.p. 115—7°C.

Preparation 3

*Methyl 4-(hydroxymethyl)-3-thiophenecarboxylate*

A solution of methyl 5-(chloromethyl)-3-thiophenecarboxylate (1.7 g) in 50% aqueous acetone (60 ml) was treated with silver nitrate (1.52 g). After 2h the resulting precipitate was filtered and the filtrate was evaporated. The residue was extracted with diethyl ether, and the ethereal extract was washed with water. Evaporation of the solvent gave an oily residue, which was chromatographed on silica with ethyl acetate-light petroleum 60—80°; (1:1) to give the *title compound* as an oil (0.3 g) b.p. 110/0.1 mm m.p. 38—40°.

Preparation 4

*5-(hydroxymethyl)-N,N-dimethyl-3-thiophenecarboxamide*

A mixture of methyl 5-(hydroxymethyl)-3-thiophenecarboxylate (3 g), sodium methoxide (0.2 g) and excess anhydrous dimethylamine in dry methanol (20 ml) was stirred for 24h at ambient temperature. The solvent was removed *in vacuo*, water was added and the product was extracted with ethyl acetate. The organic extract was evaporated to leave an oily residue which was chromatographed on silica with ethyl acetate-light petroleum 40—60°; (1:1) to give the *title compound* (1.3 g).

I.R. ($CHBr_3$) OPH 3,595 $cm^{-1}$; C = 1,620 $cm^{-1}$

Preparation 5

*4-(Dimethylaminomethyl)-2-thiophenemethanol*

A solution of 5-(hydroxymethyl)-N,N-dimethyl-3-thiophenecarboxamide (1.7 g) in dry tetrahydrofuran (150 ml) was treated with a 0.5M solution of aluminium hydride in tetrahydrofuran (26 ml). After 2h at room temperature, water (5 ml) was cautiously added and the mixture was filtered through diatomaceous earth. The filtrate was evaporated to leave an oily residue, which was dissolved in 2M hydrochloric acid (20 ml). The aqueous solution was washed with diethyl ether, basified with 5M sodium hydroxide and extracted with ethyl acetate.

The organic extract was distilled to give the *title compound* as an oil (1 g) b.p. 110/0.1 mm.

I.R. ($CHBr_3$) OH 3585 $cm^{-1}$; $Me_2NCH_2$ 2,775 $cm^{-1}$ and 2,820 $cm^{-1}$

Preparation 6

*1-(3-Methyl-2-thienylmethyl)piperidine*

A mixture of 3-methyl-2-thiophenecarboxaldehyde 20 g), formic acid (6 ml) and N-formyl piperidine (44.4 ml) was heated under reflux for 12h. Water (50 ml) and 2M hydrochloric acid (30 ml) were added; and the aqueous solution was washed with ether. The solution was basified to pH 10 with 2M sodium hydroxide (30 ml) and extracted with ether. The organic extract was evaporated to give a brown oil which was distilled to afford the *title compound* as a colourless oil (11.8 g) b.p. 70°/0.4 mm.

N.M.R. ($CDCl_3$) 2.9, d, (1H); 3.27, d, (1H); 6.48, s, (2H); 7.6, m, (4H); 7.83, s, (3H); ca 8.5, m, (>6H).

Preparation 7

*5-(Dimethylaminomethyl)-4-methyl-2-thiophenemethanol*

A solution of N,N-3-trimethyl-2-thiophenemethanamine (1.5 g) in dry tetrahydrofuran (50 ml) was treated with a solution of n-butyl lithium (1.6M; 7 ml) at room temperature, under nitrogen. After 4h, gaseous formaldehyde (excess) was added and the mixture was heated at 40°C for 20h.

Water (100 ml) and chloroform (100 ml) were added and the organic solution evaporated *in vacuo* to leave an oily residue which was dissolved in ethanol (50 ml) and treated with sodium borohydride (0.1 g), followed by acetic acid (10 ml). The reaction mixture was evaporated to dryness and the residue was dissolved in sodium carbonate solution (8%, 50 ml) and extracted with chloroform. The organic extract was distilled to give the *title compound* as a colourless oil (0.65 g) b.p. 120°/0.1 mm.

The oxalate salt was formed in and recrystallised from a mixture of ethanol and ethyl acetate m.p. 116—7°C.

(b) Similarly, 1-(3-methyl-2-thienylmethyl)piperidine (3 g) gave 4-methyl-5-(1-piperidinylmethyl)-2-thiophenemethanol as an oil (2.4 g) b.p. 120°/0.2 mm.

N.M.R. ($CDCl_3$) 3.32, s, (1H); 5.32, s, (2H); 6.48, s, (2H); 7.38, brs, (1H); 7.50—7.7, m, (4H); 7.89, s, (3H); 8.30—8.80, m. (6H).

Preparation 8

(a) *4-[[(2-(Amino)ethyl]thio]methyl-N,N-dimethyl-2-thiophenemethanamine*

A mixture of 5-(dimethylaminomethyl)-3-thiophenemethanol (1 g) and 2-aminoethanethiol

hydrochloride (0.67 g) was stirred in concentrated hydrochloric acid (7 ml) at 0°C for 2 hours; and then at room temperature for 48 hours. Solid anhydrous sodium carbonate was added and the product was extracted into ethyl acetate.

The organic extract was distilled to give the *title compound* as a colourless oil (0.75 g) b.p. 130°/0.05 mm. The oxalate sale was formed in ethanol and recrystallised from ethanol and water m.p. 178—9°.

The following compounds were similarly prepared from 2-aminoethanethiol hydrochloride (A) and the corresponding thiophenemethanol.

(b) A (1.5 g) and 5-(1-pyrrolidinylmethyl)-3-thiophenemethanol (2.4 g gave 2-[[4-(1-pyrrolidinylmethyl)-2-thienylmethyl]thio]ethanamine (1.4 g). Oxalate salt m.p. 162—4°.

(c) A (1.6 g) and 5-(1-piperidinylmethyl)-3-thiophenemethanol (2.9 g) gave 2-[[4-(1-pyrrolidinylmethyl)-2-thienylmethyl]thio]ethanamine (2.2 g), Oxalate salt m.p. 89—91°.

(d) A (0.65 g) and 4-(dimethylaminomethyl)-2-thiophenemethanol (0.9 g) fave 5-[[2-aminoethyl]-thio]methyl-N,N-dimethyl-3-thiophenemethanamine (0.8 g) b.p. 130°/0.1 mm. Oxalate salt m.p. 151—2°.

(e) A (0.6 g) and 5-(dimethylaminomethyl)-4-methyl-2-thiophenemethanol (0.6 g) gave 5-[[2-aminoethyl]thio]methyl-N,N,3-trimethyl thiophenemethanamine as a colourless oil (0.69 g) b.p. 150°/0—1 mm. Oxalate salt m.p. 212° dec.

(f) A (1.11 g) and 4-methyl-5-(1-piperidinylmethyl)thiophene-2-methanol (2.2 g) gave 2-[[4-methyl-5-(1-piperidinylmethyl)-2-thienylmethyl]thio]ethanamine as a yellow viscous oil (1.88 g) b.p. 170°/0.05 mm.

(g) A (1.2 g) and 4-(1-pyrrolidinylmethyl)-2-thiophenemethanol (1.7 g) gave 2[[4-(1-pyrrolidinylmethyl)-2-thienylmethyl]thio]ethanamine, b.p. 165°/0.02 mm. Oxalate salt m.p. 147—8°.

(h) A (1.3 g) and 5-methyl-4-(dimethylaminomethyl)-2-thiophenemethanol (2 g) gave 5-[[2-(aminoethyl)]thio]methyl-N,N,2-trimethyl-3-thiophenemethanamine (1.9 g). Oxalate salt m.p. 146—7°.

## Preparation 9

*4-(1-Pyrrolidinylmethyl)-2-thiophenemethanol*

*1-(3-Thienylcarbonyl) pyrrolidine*

A mixture of 3-thiophenecarboxylic acid (15 g) oxalylchloride (15 ml) and pyridine (0.05 ml) in toluene (100 ml) was heated at reflux for 2 hours. The excess reagent was removed *in vacuo* and the toluene solution was added to a solution of dry pyrrolidine (25 ml) in dry toluene (100 ml). After 2h the reaction mixture was washed with dilute hydrochloric acid, 8% aqueous sodium carbonate, saturated brine and water. The organic solution was dried and evaporated to give the *title compound* (1.8 g) as a white crystalline solid m.p. 70—73°.

*1-(3-Thineylmethyl)pyrrolidine*

A solution of 1-(3-thienylcarbonyl) pyrrolidine (12 g) in dry diethylether (200 ml) was added to a slurry of lithium aluminium hydride (2.6 g) in dry diethylether (100 ml). The mixture was stirred for 1 hour, and aqueous 5M sodium hydroxide (20 ml) was added. The mixture was filtered and the filtrate was distilled to give the *title compound* (9.8 g) as a colourless oil. b.p. 120/15 mm.

*4-(1-Pyrrolidinylmethyl)-2-thiophenemethanol*

A solution of paraformaldehyde (1.5 g) in *conc.* hydrochloric acid (15 ml) and acetic acid (30 ml) was treated with 1-(3-thienylmethyl)pyrrolidine (2.7 g), and the mixture was stirred for 18 hours, at room tmeperature.

The mixture was poured onto saturated aqueous sodium carbonate and extracted with chloroform. The chloroform solution was extracted with dilute hydrochloric acid. The aqueous extract was basified with anhydrous sodium carbonate and extracted with chloroform. The chloroform extract was dried and evaporated to give an oil which was purified firstly by thin layer chromatography on silica with a mixture of ethyl acetate:ethanol:0.88 ammonia (40:3:2) as eluant, and finally by distillation to give the *title compound* (0.8 g) as a colourless oil. b.p. 140/0.02 mm.

## Preparation 10

*5-(Dimethylaminomethyl)-3-thiophenemethanol.*

*Methyl 5-[(dimethylamino)carbonyl]-3-thiophenecarboxylate*

A mixture of methyl 3-thiophenecarboxylate (2.8 g) and N,N-dimethylcarbonyl chloride (2.0 ml) was treated with stannic chloride for 5 minutes and the mixture was stirred for 20 hours. Chloroform and iced water were added. The organic solution was washed with 8% aqueous sodium carbonate, dried and evaporated to give a viscous oil. This oil was heated at 70° *in vacuo* and the residue was dissolved in methyl acetate. Petroleum ether was added to give the *title compound* (1.1 g) as a crystalline solid m.p. 51—2°C.

*5-(Dimethylaminomethyl)-3-thiophenemethanol*

A solution of methyl 5-[(dimethylamino)carbonyl]-3-thiophenecarboxylate (0.5 g) in dry

diethylether (50 ml) was added to a slurry of lithium aluminium hydride (0.3 g) in dry diethylether (100 ml). The mixture was stirred for 3 hours at room temperature. Water was cautiously added, the mixture was filtered and the filtrate was evaporated to give the *title compound* (0.4 g) as a colourless oil b.p. 120°0.1 mm. The oxalate was formed in and recrystallised from ethanol m.p. 107—8°.

Preparation 11

*5-Methyl-4-(dimethylaminomethyl)-2-thiophenemethanol*

*N,N,3-trimethyl-3-thiophene carboxamide.*

A mixture of 2-methyl-3-thiophene carboxylic acid (2 g) and oxalyl chloride (2 ml) in dry toluene (20 ml) was heated under reflux and was treated with pyridine (0.05 ml). The reaction mixture was heated at reflux for 1h, cooled and partially evaporated. The residue was added to a solution of dimethylamine in toluene at 0 to 5°C. After 2 hours water and diethyl ether were added. The organic phase was washed with dilute hydrochloric acid, sodium hydroxide and water dried and evaporated to an oil which was distilled to give the *title compound* (1.4 g) as a colourless oil b.p. 100°/0.1 mm.

*N,N,2-Trimethyl-3-thiophenemethanamine*

A solution of N,N,3-trimethyl-3-thiophene carboxamide (1.3 g) in dry diethylether (50 ml) was added to a slurry of lithium aluminum hydride (0.3 g) in dry diethylether (100 ml) at room temperature. The mixture was stirred for 1 hour and then water was added. The mixture was filtered and the filtrate was evaporated to give the *title compound* (0.75 g) as a colourless oil. The picrate salt was formed in ethanol m.p. 155°.

*5-Methyl-4-(dimethylaminomethyl)-2-thiophenemethanol*

A mixture of N,N,2-trimethyl-3-thiophenemethanine (6.1 g), paraformaldehyde (3.0 g) in *conc.* hydrochloric acid (20 ml) and acetic acid (45 ml) was stirred at 0 to 5°C for 1 hour, and at 8° for 72 hours. The mixture was added to an ice-cold, saturated solution of sodium carbonate. Aqueous 5M sodium hydroxide was added and the mixture was stirred for 20 hours. The solution was extracted with chloroform and the organic extract was washed with dilute sodium hydroxide and saturated brine, dried and evaporated to leave an oil. This residue was distilled to give the *title compound* (6 g) as a pale yellow oil b.p. 120°/0.1 mm. The oxalate salt was formed in ethanol and ethyl acetate m.p. 129—131°C.

Preparation 12

*3-[5-(Dimethylaminomethyl)-3-thiophenemethoxy]propanamine dioxalate salt*

To a solution of 5-(dimethylaminomethyl)-thiophene-3-methanol (5.2 g) and potassium tertiarybutoxide (3.4 g) in dry dimethylformamide (100 ml) was added 3-chloropropylamine hydrochloride (7.8 g) in dimethylformamide (10 ml). The mixture was stirred at 20° for 3h. A further portion of potassium tertiarybutoxide (6.8 g) was added followed by 3-chloropropylamine hydrochloride (7.8 g) in dimethylformamide (10 ml) and the reaction was stirred at 20° for 18h. The mixture was concentrated *in vacuo*, tetrahydrofuran (100 ml) was added followed by potassium carbonate and the mixture was filtered. The filtrate was concentrated, and purified by column chromatography on silica using methanol and methanol:amonia (79:1) to give an oil (2.2 g). Addition of oxalic acid to a solution of oil in ethanol gave the *title compound* as an off-white solid, m.p. 176—178° (d).

| | | | |
|---|---|---|---|
| Analysis Found: | C, 43.6; | H, 5.8; | N, 6.6; |
| $C_{15}H_{24}N_2O_9S$ requires: | C, 44.1; | H, 5.9; | N, 6.9% |

Preparation 13

*Dimethyldithio-N-phenacylcarbonimidinothioate*

A suspension of potassium carbonate (14.1 g) and dimethyl dithiocarbonimidinothioate hydro-iodide (10.2 g) in acetone (150 ml) was stirred at 20° during 2h. Phenylacetyl chloride (6.8 g) was added dropwise and stirring was continued for 17 h at 20°. The mixture was partially evaporated, diluted with water (300 ml) and extracted with ethyl acetate. The combined organic extracts were evaporated to give the *title compound* as a pale green oil (7.9 g) b.p. 160—164°/0.06 mm.

T.l.c. ethyl acetate $R_f$ 0.8.

Preparation 14

*4-[3-(Amino)propoxy]-N,N-dimethyl-2-thiophenemethanamine*

4-Bromo-N,N-dimethyl-2-thiophenemethanamine

A mixture of 4-bromo-2-thiophenecarboxaldehyde (4.8 g), dry dimethylformamide (4.3 ml) and 98% formic acid (1.4 ml), was heated at 120° for 30h. The cooled mixture was poured into water (100 ml) and basified with anhydrous sodium carbonate. Diethyl ether (2 × 100 ml) was added and the ethereal solution was extracted with dilute hydrochloric acid (50 ml). The aqueous solution was basified

with anhydrous sodium carbonate and extracted with diethyl ether (200 ml). The ethereal extracts were distilled to give the *title compound* (2.5 g) as a colourless oil, b.p. 70°/0.1 mm.

*3-[5-(Dimethylaminomethyl)-3-thienyloxy]-1-propanol*

A mixture of 1,3-propanediol (20 ml) and sodium hydride (1 g) was stirred at 80° for 1h. Copper (II) oxide (1.3 g), sodium iodide (0.05 g) and 4-bromo-N,N-dimethyl-2-thiophenemethanamine (5 g) were added and heating was continued for 2 days. Dilute hydrochloric acid (60 ml) was added and the aqueous solution was washed with diethyl ether (50 ml), basified with aqueous sodium hydroxide and extracted with chloroform (200 ml). The organic extract was distilled to give the *title compound* (1.7 g) as an off-white wax. m.p. 32—4°.

*3-[5-(Dimethylaminomethyl)-3-thienyloxy]-1-propanol methanesulphonate hydrochloride*

A solution of 3-[5-(dimethylaminomethyl)-3-thienyloxy]-1-propanol (0.25 g) and methanesulphonyl chloride (0.1 ml) in dry dichloromethane (10 ml) at 0—5° was stirred for 1h. The mixture was poured onto an ice cold 4% aqueous solution of sodium carbonate (50 ml). The solution was extracted with dichloromethane (20 ml). The organic extract was evaporated to give the *title compound* (0.05 g) as a white solid.

I.R. (Nujol) 2,550; 2,450, 1,350, 1170 and 1,195 $cm^{-1}$

N.M.R. ($CDCl_3$) of base: 3.40, brs (1H), 3.82, d, (1H); 5.62, t, (2H); 5.98, t, (2H); 6.50, s, (2H); 7.01, s, (3H); 7.75—7.80, s+m, (8H).

*2-[3-[[5-(Dimethylaminomethyl)-3-thienyloxy]propyl]]-(1H)-isoindole-1,3-(2H) dione*

A solution of 3-[5-(dimethylaminomethyl)-3-thienyloxy]-1-propanol methanesulphonate hydrochloride (6 g) in dichloromethane (200 ml) was washed with dilute sodium hydroxide (50 ml). The organic solution was dried ($Na_2SO_4$), filtered and evaporated leaving an oil. This oil was dissolved in dry dimethylformamide (100 ml), and potassium phthalimide (3.7 g) was added. The mixture was stirred for 72h. The excess solvent was removed *in vacuo* and the residue was dissolved in diethylether (1 l) and washed with water (2 l). The ethereal solution was dried ($Na_2SO_4$), filtered and evaporated to give the *title compound* (4.8 g) as an amber oil.

I.R. ($CHBr_3$) 2,780, 2,820, 1,770, 1,710$cm^{-1}$.

*4-[3-(Amino)propoxy]-N,N-dimethyl-2-thiophenemethanamine*

A solution of 2-[3-[[5-(dimethylaminomethyl)-3-thienyloxy]propyl]]-(1*H*)-isoindole-1,3(2H)-dione (4.6 g) was dissolved in a 30% solution of methylamine in ethanol (100 ml). The mixture was stirred at room temperature for 4h and heated on a steam-bath for 2h. The solvent was evaporated and the residue was dissolved in dilute hydrochloric acid (20 ml). The aqueous solution was basified with anhydrous sodium carbonate, washed with diethyl ether (150 ml) and extracted with toluene (200 ml). The toluene solution was distilled to give the *title compound* (1.6 g) as a colourless oil, b.p. 120°/0.06 mm.

The oxalate salt was formed in and recrystallised from ethanol and water m.p. 150—2°.

Preparation 15

*5-[(3-Aminopropyl)thio]-N,N,3-trimethyl-2-thiophenemethanamine*

*N-[3-[[5-(Dimethylamino)methyl-4-methyl-2-thienyl]thio]propyl]-isoindole-1,3(2H)-dione*

A solution of N,N,3-trimethyl-2-thiophenemethanamine (6.2 g) in dry tetrahydrofuran (100 ml) was treated with a solution of n-butyl lithium in hexane (1.6M, 27.5 ml) at 0°, under nitrogen. After 1h, sulphur (1.4 g) was added. After a further 0.5h a solution of N-(3-bromopropyl)-isoindole-1,3(2H)-dione (11.8 g) in dry tetrahydrofuran (50 ml) was added and the mixture was stirred at room temperature for 18h. Water (100 ml) was added and the mixture extracted with ether. The organic extract was evaporated to give the *title compound* as a yellow oil (15.3 g).

I.R. ($CHBr_3$) 2820, 2775, 1770, 1705, 1392 $cm^{-1}$

*5-[(3-Aminopropyl)thio]-N,N,3-trimethyl-3-thiophenemethanamine*

N-[3-[[5-(Dimethylamino)methyl-4-methyl-2-thienyl]thio]propyl]isoindole-1,3(2H)-dione (15.2 g) and hydrazine hydrate (8 ml) were stirred at room temperature in tetrahydrofuran (150 ml) for 48 h. The mixture was filtered and the filtrate was distilled to give the *title compound* as an orange oil (6.9 g) b.p. 160—170°/0.04 mm.

Preparation 16

*5-[4-[(3-Hydroxymethyl-1-methyl-1H-1,2,4-triazol-5-yl)amino]butyl]-3-methyl-2-thiophenecarboxaldehyde*

*2-(3-Methyl-2-thienyl)-1,3-dioxolane*

3-Methyl-2-thiophenecarboxaldehyde (12 g), 1,2-ethanediol (20 ml) and a trace of toluene-*p*-

sulphonic acid were heated at reflux in toluene (250 ml) under Dean and Stark conditions for 4h. The reaction mixture was cooled and washed with 5% aqueous sodium carbonate solution and water. The toluene solution was evaporated to give an oil which was distilled to give the *title compound* as a pale yellow oil (14.5 g), b.p. 95°/0.1 mm.

*2-[5-(4-Bromobutyl)-3-methyl-2-thienyl]-1,3-dioxolane*

A solution of 2-(3-methyl-2-thienyl)-1,3-dioxolane (12.3 g) in dry tetrahydrofuran (150 ml) was treated with a solution of n-butyl lithium in hexane (1.6M, 50 ml) at —60°, under nitrogen. After 1h, 1,4-dibromobutane (17.3 g) was added and the mixture was stirred at —50° for 3h and at room temperature for 18h. Water (75 ml) was added and the mixture extracted with ether. The organic extract was distilled to give the *title compound* as a light brown oil (15.1 g) b.p. 250°/0.15 mm

*N-N-[[5-(1,3-Dioxolan-2-yl)-4-methyl-2-thienyl]-4-butyl]-isoindole-1,3(2H)-dione*

2-[5-(4-Bromobutyl)-3-methyl-2-thienyl]-1,3-dioxolane (13.3 g) and potassium phthalimide (8.9 g) were stirred at 50° in dry dimethylformamide (130 ml) for 4h and then at room temperature for 18h. The mixture was poured into water (1500 ml) and extracted with ether. The organic extract was evaporated to give the *title compound* as a light yellow oil (16.1 g)

I.R. ($CHBr_3$) 1770, 1710, 1395, 1070, 1033, 720 $cm^{-1}$

*4-[5-(1,3-Dioxolane-2-yl)-4-methyl-2-thienyl]-butanamine*

N-[[5-(1,3-Dioxolan-2-yl)-4-methyl-2-thienyl]-4-butyl]-isoindole-1,3(2H)-dione (16.1 g) and hydrazine hydrate (8 ml) were stirred at room temperature in tetrahydrofuran (150 ml) for 24h. The mixture was filtered and the filtrate was distilled to give the *title compound* as a red-brown oil (5.34 g), b.p. 230°/0.1 mm.

*5-[4-[(3-Hydroxymethyl-1-methyl-1H-1,2,4-triazole-5-yl)-amino]butyl]-3-methyl-2-thiophene-carboxaldehyde*

A mixture of 4-[5-(1,3-dioxolan-2-yl)-4-methyl-2-thienyl]-butanamine (2.73 g) and methyl N-[2-(acetoxy)-acetyl]-1-methyl-2-(phenylmethylene)-hydrazinecarboximidothioate (3.38 g) was heated at 60° for 2h. Toluene (20 ml) and 5N hydrochloric acid (12 ml) were added and the mixture stirred at room temperature for 18h. The mixture was basified to pH 8, washed with toluene, basified to pH 10 and extracted with ethyl acetate. The extract was evaporated to give an oil which was purified by column chromatography on silica using 3:1 ethyl acetate;methanol as eluant to give the *title compound* as a pale yellow solid (0.6 g) m.p. 118—121° (softens).

Example 1

(a) *N-Methyl-N'-[2-[[5-dimethylaminomethyl)-3-thienylmethyl]-thio]ethyl]-2-nitro-1,1-ethene-diamine*

A mixture of 4-[[2-(amino)ethyl]thio]methyl-N,N-dimethyl-2-thiophene methanamine (0.35 g) and N-methyl-1-(methylthio)-2-nitroethenamine (0.23 g) in water (5 ml) was stirred at room temperature for 24 hours. The resulting precipitate was collected by filtration and recrystallised from methanol and water to give the *title compound* as an off-white solid (0.27 g) m.p. 143°.

| | | | |
|---|---|---|---|
| Asssay found:— | C, 47.3; | H, 6.7; | N, 16.8% |
| $C_{13}H_{22}N_4O_2S_2$ requires: | C, 47.3; | H, 6.7; | N, 17.0% |

The following compounds were similarly prepared from N-methyl-1-(methylthio)-2-nitroethen-amine [A] and the appropriate diamine.

(b) A (0.45 g) and 2-[[4-(1-pyrrolidinylmethyl)-2-thienyl methyl]thio]ethanamine (0.75 g) gave N-Methyl-N'-[2-[[5-(1-pyrrolidinylmethyl)-3-thienylmethyl]thio]ethyl]-2-nitro-1,1-ethenediamine (0.72 g), m.p. 128—130°.

| | | | |
|---|---|---|---|
| Assay found: | C, 50.4; | H, 6.7; | N, 15.15; |
| $C_{15}H_{24}N_4O_2S_2$ requires; | C, 50.5; | H, 6.8; | N, 15.7% |

(c) A (0.23 g) and 5-[[2-(amino)ethyl]thio]methyl-N,N-dimethyl-3-thiophenemethanamine (0.35 g) gave N-methyl-N'-[2-[[4-(dimethylaminomethyl)-2-thienylmethyl]thio]ethyl]-2-nitro-1,1-ethene-diamine, carbonate salt as a white solid (0.2 g) m.p. 99—100°.

| | | | |
|---|---|---|---|
| Assay found: | C, 44.93; | H, 6.46; | N, 15.45 |
| $C_{13}H_{22}N_4O_2S_2 \cdot \frac{1}{2}H_2CO_3$ requires: | C, 44.85; | H, 6.41; | N, 15.50% |

(d) A (0.28 g) and 2-[[5-(1-piperidinylmethyl)-3-thienylmethyl]thio]ethanamine (0.5 g) gave N-

methyl-N'-[2-[[5-(1-piperidinylmethyl)-3-thienylmethyl]thio]ethyl]-2-nitro-1,1-ethenediamine as a white solid (0.4 g) m.p. 101—3°.

| | | | |
|---|---|---|---|
| Assay found:— | C, 51.93; | H, 7.03; | N, 15.09 |
| $C_{16}H_{26}N_4O_2S_2$ requires | C, 51.87; | H, 7.02; | N, 15.13% |

(e) A (0.2 g) and 5-[[[2-(amino)ethyl]thio]methyl]-N,N,3-trimethylthiophenemethanamine (0.3 g) gave N-[2-[[5-Dimethylaminomethyl)-4-methyl-2-thienylmethyl]thio]ethyl-N'-methyl-2-nitro-1,1-ethenediamine (which was purified by column chromatography on silica using methanol as eluant) as a white solid (0.38 g) m.p. 82—3°.

| | | | |
|---|---|---|---|
| Assay found: | C, 48.6; | H, 7.0; | N, 15.9 |
| $C_{14}H_{24}O_2S_2$ requires | C, 48.8; | H, 7.0; | N, 16.3% |

(f) A (0.27 g) and 2-[[4-methyl-5-(1-piperidinylmethyl)-2-thienylmethyl]thio]ethanamine (0.5 g) gave N-methyl-N'-[2-[[4-methyl-5-(1-piperidinylmethyl)-2-thienylmethyl]thio]ethyl]-2-nitro-1,1-ethenediamine as a white solid, recrystallised from methyl acetate-light petroleum b.p. 60—80° (1:3), (0.1 g) m.p. 81°.

| | | | |
|---|---|---|---|
| Assay found:— | C, 53.11; | H, 7.38; | N, 14.52 |
| $C_{17}H_{28}N_4O_2S_2$ requires | C, 53.11; | H, 7.34; | N, 14.58% |

(g) A (0.25 g) and 2-[[4-(1-pyrrolidinylmethyl)-2-thienylmethyl]thio]ethanamine (0.4 g) gave N-Methyl-N'-[2-[[4-(1-pyrrolidinylmethyl)-2-thienylmethyl]thio]ethyl]-2-nitro-1,1-ethenediamine as a white solid (0.25 g) m.p. 109—111°.

| | | | |
|---|---|---|---|
| Assay found: | C, 50.25; | H, 6.75; | N, 15.4 |
| $C_{15}H_{24}N_4O_2S_2$ requires | C, 50.55; | H, 6.8: | N, 15.7 |

(h) A (0.26 g) and 5-[[2-(amino)ethyl]thio]methyl-N,N,2-trimethyl-3-thiophenemethanamine (0.4 g) gave N-Methyl-N'-2-[[4-(dimethylaminomethyl)-5-methyl-2-thienylmethyl]thio]ethyl]-2-nitro]-1,1-ethenediamine as a white solid (0.25 g) m.p. 100—101°.

| | | | |
|---|---|---|---|
| Assay found: | C, 48.65; | H, 6.85; | N, 16.10 |
| $C_{14}H_{24}N_4O_2S_2$ requires | C, 48.80; | H, 7.0; | N, 16.25 |

(i) A (0.27 g) and 3-[5-(dimethylaminomethyl)-3-thiophenemethoxy]propanamine (0.4 g) gave N-[3-[5-(dimethylaminomethyl)-3-thienylmethoxy]propyl]-N'-methyl-2-nitro-1,1-ethenediamine (0.42 g) as a pale orange oil.

T.l.c. System B Rf 0.43

N.M.R. ($CDCl_3$) 0.0, br, (1H); 3.9, br, (1H); 2.88, s, (1H); 3.12, s, (1H); 3.45, s, (1H); 5.59, s, (2H); 6.3, m, (2H); 6.43, s, (2H); 6.7, m, (2H); 7.40, d, (3H); 7.75, s, (6H); 8.08, m, (2H).

(j) A (0.35 g) and 4-[3-(amino)propoxy]-N,N-dimethyl-2-thiophenemethanamine (0.5 g) gave N-[3-[5-(dimethylaminomethyl)-3-thienyloxy]propyl]-N'-methyl-2-nitro-1,1-ethenediamine (0.35 g), which was purified by column chromatography and crystallised from methyl acetate m.p. 113°

N.M.R. ($CDCl_3$) —0.4, brs, (1H); 3.40, brs, (3H); 3.82, d, (1H); 6.00, t, (2H); 6.50—6.55, s+brs, (4H), 7.1, brs, (3H); 7.75—7.9, s+m, (8H).

(k) A (0.4 g) and 5-[(3-aminopropyl)thio]-N,N,3-trimethyl-2-thiophenemethanamine gave N-[3-[[5-(dimethylamino)methyl-4-methyl-2-thienyl]thio]propyl]-N'-methyl-2-nitro-1,1-ethenediamine which was purified by column chromatography as a light brown oil (0.353 g).

N.M.R. ($CDCl_3$): —0.25 br.s. (1H); 3.18, s, (1H); 3.4, br.s, (1H) overlain by 3.4, s, (1H); 6.53, s, (2H); 6.55, q, (2H); 7.1, m, (3H); 7.18, t, (2H); 7.72, s, (6H); 7.9, s, (3H); 8.05, m, (2H).

| | | | |
|---|---|---|---|
| Found: | C, 48.8; | H, 7.0; | N, 16.2; |
| $C_{14}H_{24}N_4O_2S_2$ requires: | C, 48.8; | H, 7.1; | N, 15.8%. |

Example 2

(a) *$N^5$-[2-[[5-(Dimethylaminomethyl)-3-thienylmethyl]thio]ethyl]-1-methyl-1H-1,2,4-triazole-3,5-diamine*

A mixture of 4-[[2-(amino]ethyl]thio]methyl-N,N-dimethyl-2-thiophenemethanamine (0.375 g) and methyl N-cyano-1-methyl-2-(phenylmethylene)hydrazine carboximidothioate (0.35 g) was heated at 60° *in vacuo* for 1 hour. The resulting oil was dissolved in toluene (30 ml) and 2M hydrochloric acid (2 ml) was added.

After 3 hours at room temperature 8% aqueous sodium bicarbonate (20 ml) was added and the resulting aqueous solution was washed with toluene. The aqueous solution was further basified with 5M sodium hydroxide (30 ml) and the product was extracted with ethyl acetate. The organic solution was evaporated to give the *title compound* as an amber oil (0.3 g).

N.M.R. ($CDCl_3$) 3.0, s, (1H); 3.1, s, (1H); 5.6, t, (1H); 6.0, brs, (2H); 6.4, s, (2H); 6.5, s, (2H); 6.6, s+q, (5H); 7.4, t, (2H); 7.8, t, (6H).

T.l.c. System A Rf 0.5. The following compounds were similarly prepared from methyl N-cyano-1-methyl-2-(phenylmethylene)hydrazinecarboximidothioate (A) and the appropriate diamine.

(b) A (0.42 g) and 5-[[2-(amino)ethyl]thio]methyl-N,N-dimethyl-3-thiophenemethanamine (0.4 g) gave $N^5$-[2-[[4-(dimethylaminomethyl)-2-thienylmethyl]thio]ethyl]-1-methyl-1H-1,2,4-triazole-3,5-diamine (0.3 g).

| | | | |
|---|---|---|---|
| Assay found:— | C, 45.3; | H, 7.1; | N, 24.1 |
| $C_{13}H_{22}N_6S_2H_2O$ requires | C, 45.4; | H, 7.0; | N, 24.4% |

N.M.R. ($CDCl_3$) 2.98, 2s (2H); 5.4—5.9, br, (3H); 6.13, s, (2H); 6.55, 6.61, 2s+m, (7H); 7.28, b, (2H); 7.7, s, (6H).

(c) A (0.43 g) and 2-[[4-(1-pyrrolidinylmethyl)-2-thienylmethyl]thio]ethanamine (0.45 g) gave $N^5$-[2-[[5-(1-pyrrolidinylmethyl)-3-thienylmethyl]thio]ethyl]-1-methyl-1H-1,2,4-triazole-3,5-diamine as an amber gum (0.4 g).

| | | | |
|---|---|---|---|
| Assay found: | C, 49.79; | H, 6.81; | N, 22.83 |
| $C_{15}H_{24}N_6S_2{\cdot}H_2O$ requires | C, 49.82; | H, 6.97; | N, 23.24% |

N.M.R. ($CDCl_3$) 3.0—3.1, 2s, (2H); 5.6, brt, (1H); 6.05, br, (2H); 6.23, s, (2H); 6.33, s, (2H); 6.52, s+m, (5H); 7.31, t, (2H); 7.4, m, (4H); 8.25, m, (4H).

(d) A (0.3 g) and 5-[[2-(amino)ethyl]thio]methyl]-N,N,3-trimethyl-2-2-thiophenemethanamine (0.3 g) gave 1-methyl-$N^5$-[2-[[5-[(dimethylamino)methyl-4-methyl-2-thienylmethyl]thio]ethyl]-1H-1,2,4-triazole-3,5-diamine as an amber gum (0.15 g).

T.l.c. System A $R_f$ 0.5

N.M.R. ($CDCl_3$) 3.4, s, (1H); 5.7—6.1, t+brs+s, (5H); 6.5—6.6, s+s+m, (7H); 7.2, m, (2H); 7.8, s, (6H); 7.9, s, (3H).

## Example 3

*N-[[[2-[[5-(Dimethylaminomethyl)-3-thienylmethyl]thio]ethyl]amino] (methylamino)methylene]-methanesulphonamide*

A mixture of 4-[[2-(amino)ethyl]thio]methyl-N,N-dimethyl-2-thiophenemethanamine (0.4 g) and S,S'-dimethyl-N-(methylsulphonyl)-dithio-carbonimidic acid (0.35 g) in ethanol (10 ml) was heated at 60°C for 1 hour. A 33% solution of methylamine in ethanol (10 ml) was added and the mixture was stirred for 20h. The solvent was evaporated *in vacuo* and the residue was purified by column chromatography on silica using methanol to give the *title compound* (0.5 g) as an amber gum.

| | | | |
|---|---|---|---|
| Assay found:— | C,41.0; | H, 6.6; | N, 14.7 |
| $C_{13}H_{24}N_4O_2S_3$ requires | C, 40.8; | H, 6.9; | N, 14.5% |

N.M.R. ($CDCl_3$): 2.9, s, (1H); 3.09, s, (1H); 3.25 brs, (1H); 4.30, brs. (1H); 6.28, s, (2H); 6.39, s, (2H); 6.61, q, (6.61); 7.08, s, (3H); 7.16, d, (3H); 7.36, t, (2H); 7.73, s, (6H).

## Example 4

*$N^{11}$-Cyano-N-[2-[[5-[(dimethylamino)methyl]-4-methyl-2-thienylmethyl]thio]ethyl]-$N^1$-methyl-guanidine*

5-[[2-(Amino)ethyl]thio]methyl-N,N,3-trimethylthiophenemethanamine (0.5 g) and methyl N-cyano-N'-methylcarbonimidothioate (0.27 g) were heated at 110—130° *in vacuo* for 7h. The resulting mixture was purified by column chromatography on silica using methanol to give the *title compound* as a brown oil (0.35 g).

T.l.c. System A Rf. 0.52

N.M.R. ($CDCl_3$) 3.57, s, (1H); 4.16, br.q, (1H); 4.55, br, t, (1H); 6.17, s, (2H); 6.52, s, (2H); 6.63, q, (2H); 7.17, s, (3H); 7.30, t, (2H); 7.75, s, (6H); 7.9, s, (3H).

## Example 5

(a) *1-Methyl-5-[[2-[[5-(Dimethylaminomethyl)-3-thienylmethyl]thio]ethyl]amino]-1H-1,2,4-triazole-3-methanol dihydrochloride*

A mixture of 4-[[2-(amino)ethyl]thio]methyl-N,N-dimethyl-2-thiophenemethanamine (0.4 g) and methyl N-[2-(acetoxy)acetyl]-1-methyl-2-(phenylmethylene)-hydrazinecarboximidothioate (0.56 g) was heated at 60° for 1h. Toluene (25 ml) and dilute hydrochloric acid (3 ml) were added and the mixture

was stirred for 3h at room temperature and then heated for 30 min. on a steam-bath. Anhydrous sodium bicarbonate was added and the mixture was washed with toluene. The aqueous solution was basified with sodium hdyroxide and extracted with ethyl acetate. The ethyl acetate extract was dried [$Na_2SO_4$] filtered, and evaporated to give an oil, which was purified by column chromatography on silica using methanol to give a gum. This gum was dissolved in methyl acetate (10 ml) and treated with 8% ethereal hydrogen chloride (5 ml). The resulting solid was crystallised from ethanol to give the *title compound* (0.2 g) as white needles m.p. 182—4°C.

N.M.R. ($D_2O$) 2.50, 2.66, 2xm, (2H); 5.35, s, (2H); 5.50, s, (2H); 6.19, s, (2H; 5.50, s, (2H); 6.19, s, (2H); 6.38—6.42, s+t, (5H); 7.11—7.20, s+t, (8H).

The following compounds were similarly prepared from methyl N-[2-(acetoxy)acetyl]-1-methyl-2-(phenylmethylene)-hydrazinecarboximidothioate [B] and the appropriate diamine.

(b) [B] (1.01 g) and 2-[[5-(dimethylaminomethyl)-4-methyl]-2-thienylmethyl]thio]ethanamine (0.8 g) gave 5-[[2-[[[5-(dimethylaminomethyl)-4-methyl]-2-thienylmethyl]thio]ethyl]amino]-1-methyl-1H-1,2,4-triazole-3-methanol (0.73 g) as a pale yellow oil.

N.M.R. ($CDCl_3$) 3.36, s, (1H); 5.45, s+t, (3H); 6.18, s, (2H); 6.46, s+m, (8H); 7.16, t, (2H); 7.72, s, (6H); 7.88, s, (3H).

The free base was converted to its citrate salt in dry ethyl acetate.

m.p. 45—50° (softens).

| | | | |
|---|---|---|---|
| Analysis Found: | C, 44.3: | H, 6.1; | N, 11.5; |
| $C_{15}H_{25}N_5OS_2C_6H_8O_7{\cdot}H_2O$ requires: | C, 44.6; | H, 6.2; | N, 11.4% |

(c) [B] (0.62 g) and 2-[[4-(1-pyrrolidinylmethyl]-2-thienylmethyl]thio]ethanamine (0.51 g) gave 1-methyl-5-[[2-[[[4-(1-pyrrolidinylmethyl)]-2-thienylmethyl]thio]ethyl]amino]-1H-1,2,4-triazole-3-methanol (0.24 g) as a pale yellow gum.

| | | | |
|---|---|---|---|
| Analysis Found: | C, 51.9; | H, 6.9; | N, 18.4; |
| $C_{16}H_{25}N_5OS_2 0.25\ H_2O$ requires: | C, 51.65; | H, 6.91; | N, 18.83% |

N.M.R. ($CDCl_3$) 3.05, br.s, (2H); 5.30, t, (1H); 5.52, s, (2H); 6.20, s, (2H); 6.55, m, (7H); 7.32, t, (2H); 7.55, m, (4H); 8.30, m, (4H).

(d) [B] (0.70 g) and 2-[[[4-(Dimethylaminomethyl)-5-methyl]-2-thienylmethyl]thio]ethanamine (0.5 g) gave 1-methyl-5-[[2-[[[4-(dimethylaminomethyl)-5-methyl]-2-thienylmethyl]thio]ethyl]amino]-1*H*-1,2,4-triazole-3-methanol (0.22 g) as a brown gum.

| | | | |
|---|---|---|---|
| Analysis Found: | C, 50.3; | H, 7.1; | N, 20.2; |
| $C_{15}H_{25}N_5OS_2$ requires: | C, 50.7; | H, 7.1; | N, 19.7% |

N.M.R. ($CDCl_3$) 3.13, s, (1H); 5.25—5.50, br.t, s, br.s, (4H); 6.23, s, (2H); 6.50—6.72, s, q, s, (7H); 7.30, t, (2H); 7.70—7.78, 2, xs, (9H)

(e) [B] (1.01 g) and 3-[[5-(Dimethylaminomethyl)]-3-thienylmethoxy]propanamine (0.75 g) gave 5-[[3-[[5-(dimethylaminomethyl)]-3-thienylmethoxy]propyl]amino]-1*H*-1,2,4-triazole-3-methanol (0.4 g) as a pale yellow oil.

T.l.c. System B Rf 0.54

| | | | |
|---|---|---|---|
| Analysis Found: | C, 50.4; | H, 7.3; | N, 19.1; |
| $C_{15}H_{25}N_5O_2S{\cdot}1\ H_2O$ requires: | C, 50.4; | H, 7.6; | N, 19.6% |

(f) [B] (492 mg) and 2-[[[5-(1-Piperidinylmethyl)]-3-thienylmethyl]thio]ethanamine (417 mg) gave 1-methyl-5-[[2-[[[5-(1-Piperidinylmethyl)]-3-thienylmethyl]thio]ethyl]amino]-1H-1,2,4-triazole-3-methanol (500 mg) as a pale yellow gum.

T.l.c. 1:1 ethyl acetate-methanol, Rf 0.3

N.M.R. ($CDCl_3$) 3.02, d, (1H); 3.12, d, (1H); 5.46, s, (2H); 5.5, t, (1H); 6.0, br.s. (1H); 6.36, s, (2H); 6.4, s, (2H), 6.5, s, (3H); 6.5, q, (2H); 7.3, t, (2H); 7.6, m, (4H); 8.5, m, (6H).

(g) [B] (0.57 g) and 2-[[4-Methyl-[5-(1-piperidinylmethyl)]-2-thienylmethyl]thio]ethanamine (0.50 g) gave 1-methyl-5-[[2-[[[4-methyl-5-(1-piperidinylmethyl)]-2-thienylmethyl]thio]ethyl]amino]-1*H*-1,2,4-triazole-3-methanol (0.12 g) as a cream coloured solid, m.p. 55°.

N.M.R. ($CDCl_3$) 3.4, s, (1H); 5.47—5.58, t&s, (3H); 6.2, s, (2H); 6.48—6.8, 2xs, q.br.s., (8H); 7.2, m, (2H); 7.6, m, (4H); 7.9, s, (3H); 8.5, m, (6H).

(h) B (0.75 g) and 4-[3-(amino)propoxy]-N,N-dimethyl-2-thiophenemethanamine (0.5 g) gave 5-[3-[[5-[(dimethylamino)methyl]-3-thienyloxy]propyl]amino]-1-methyl-1*H*-1,2,4-triazole-3-methanol (0.2 g) m.p. 99—100°

T.l.c. System B Rf 0.55

(i) B (0.905 g) and 5-[(3-aminopropyl)thio]-N,N,3-trimethyl-2-thiophenemethanamine (0.72 g) gave 5-[3-[[5-[(dimethylamino)-methyl]-4-methyl-2-thienyl]thio]propyl]amino-1-methyl-1*H*-1,2,4-triazole-3-methanol (0.35 g) as a pale yellow oil.

T.l.x. system B Rf 0.6

N.M.R. ($CDCl_3$) 3.18, s, (1H); 5.45, s, (2H); 5.60, s+br.t, (2H); 6.50, s, (3H), 6.52, q, (2H); 6.54, s, (2H); 7.19, t, (2H); 7.75, s, (6H); 7.90, s, (3H); 8.10, m, (2H):

## Example 6

(a) *5-[[2-[[5-(Dimethylaminomethyl)-3-thienyl]methyl]thio]ethyl]amino-3-phenylmethyl-1H-1,2,4-triazole*

To dimethyl dithio-N-phenacylcarbonimidinothioate (1.18 g) in tetrahydrofuran (20 ml) was added 4-[[2-(amino)ethyl]thio]methyl-N,N-dimethyl-2-thiophenemethanamine (1.13 g) in tetrahydrofuran (10 ml) and the reaction mixture stirred at 20° for 5h. Hydrazine hydrate (2.5 g) was added and the mixture was stirred overnight at 20°. 2N Hydrochloric acid (10 ml) was added and after stirring for 3h at 20°, the acidic layer was basified with potassium carbonate and extracted with ethyl acetate. The ethyl acetate extracts were evaporated and the residual oil purified by column chromatography on silica using chloroform followed by chloroform:methanol (19:1) to give the *title compound* (0.2 g) as a white solid m.p. 113° after recrystallisation from a mixture of ethyl acetate and light petroleum, b.p. 60—80°

| | | | |
|---|---|---|---|
| Analysis Found: | C, 58.6; | H, 6.5; | N, 17.9; |
| $C_{19}H_{25}N_5S_2$ requires: | C, 58.9; | H, 6.5; | N, 18.1% |

(b) Similarly prepared from 5-[[2-(amino)ethyl]thio]methyl-N,N,3-trimethyl-2-thiophenemethanamine (1.0 g), dimethyl dithio-N-phenacylcarbonimidinothioate (0.98 g) and hydrazine hydrate (2.1 g) was 5-[[2-[[[5-(dimethylaminomethyl-4-methyl-2-thienyl]methyl]thio]ethyl]amino]-3-phenyl-methyl-1*H*-1,2,4-triazole (0.2 g) as a white solid m.p. 116—117° (d)

| | | | |
|---|---|---|---|
| Analysis Found: | C, 59.7; | H, 6.9; | N, 17.2; |
| $C_{20}H_{27}N_5S_2$ requires: | C, 59.8; | H, 6.8; | N, 17.4% |

## Example 7

(a) *N-[5-[[2-[[[5-[(Dimethylamino)methyl]-4-methyl-2-thienyl]methyl]thio]ethyl]amino]-1-methyl-1H-1,2,4-triazol-3-yl]acetamide*

A solution of $N^5$-[2-[[[5-[(dimethylamino)methyl]-4-methyl]-2-thienyl]methyl]thio]ethyl]-1-methyl-1H-1,2,4-triazole-3,5-diamine (1.56 g) and acetic anhydride (0.52 g) in pyridine (20 ml) was stirred at room temperature for 24h. The solution was evaporated to dryness *in vacuo* and the residual yellow gum was dissolved in water (60 ml). The solution was extracted with ethyl acetate, and to the aqueous fraction was added anhydrous sodium carbonate (3 g). The solution was extracted with ethyl acetate (3 × 50 ml). The organic extracts were evaporated *in vacuo* to give the *title compound* (1.17 g) as a yellow gum.

| | | | | |
|---|---|---|---|---|
| Analysis Found: | C 50.0; | H, 6.9; | N, 21.7; | S, 16.8; |
| $C_{16}H_{26}N_6OS_2$ requires: | C 50.2; | H, 6.9; | N, 22.0; | S, 16.8% |

N.M.R. ($CDCl_3$) 1.0, br, (1H); 3.4, s, (1H); 5.2, t, (1H); 6.2, s, (2H); 6.5, s, 6.55, s, *ca*. 6.5, s, (7H); 7.27, t, (2H); 7.75, m, 7.9, s, (12H).

(b) In a similar manner, 5-[2-[[5-[(dimethylamino)methyl]-3-thienylmethyl]thio]ethyl]-1-methyl-1*H*-1,2,4-triazole-3,5,diamine (1.15 g) and acetic anhydride (0.35 ml) gave N-[5-[[2-[[5-[(dimethylamino)methyl]-3-thienylmethyl]thio]ethyl]amino]-1-methyl-1*H*-1,2,4-triazole-3-yl]-acetamide as a yellow foam (1.1 g).

T.l.c. System A Rf 0.53

N.M.R. ($CDCl_3$) 1.6, br, (1H); 2.98, d, (1H); 3.1, d, (1H); 5,47, t, (1H); 6.3, s, (2H); 6.42, s, (2H); 6.47, s, (3H); 6.53, q, (2H); 7.3, t, (2H); 7.7, m. (9H).

Examples of Pharmaceutical Compositions

*TABLETS:*

| | mg/tablet |
|---|---|
| Active ingredient | 100.00 |
| Microcrystalline Cellulose BPC | 198.50 |
| Magnesium stearate BP | 1.50 |
| Compression weight | 300.00 |

The active ingredient is sieved through a 250 $\mu$m sieve, blended with the excipients and compressed using 9.5 mm punches. Tablets of other strengths may be prepared by altering the compression weight and using punches to suit.

The tablets may be film coated with suitable film forming materials, e.g. methyl cellulose or hydroxypropyl methyl cellulose using standard techniques. Alternatively the tablets may be sugar coated.

Injection for Intravenous Administration

| | % w/v |
|---|---|
| Active ingredient | 1.00 |
| Water for injections B.P. to | 100.00 |

Sodium chloride may be added to adjust the tonicity of the solution and the pH may be adjusted to that of maximum stability and/or to facilitate solution of the active ingredient using either dilute acid or alkali.

The solution is prepared, clarified and filled into appropriate sized ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen.

Example 8

(a) *5-[[2-[[5-[[(2-Furanylmethyl)amino]methyl]-3-thienylmethyl]thio]ethyl]amino]-1-methyl-1H-1,2,4-triazole-3-methanol*

*4-[[[2-[(3-Hydroxymethyl-1-methyl-1H-1,2,4-triazol-5-yl)amino]ethyl]thio]methyl]-N,N,N-trimethyl-2-thiophenemethanium iodide*

To a solution of 5-[[2-[[5-(dimethylaminomethyl)-3-thienylmethyl]thio]ethyl]amino]-1-methyl]-1H-1,2,4-triazole-3-methanol (3.72 g) in acetone (50 ml) was added a solution of methyl iodide (1.94 g) in acetone (10 ml). After stirring at room temperature for 3h, the oil which separated was triturated to give the *title compound* (4.65 g) m.p. 146—149°

*5-[[2-[[5-[[(2-Furanylmethyl)amino]methyl]-3-thienylmethyl]thio]ethyl]amino]-1-methyl-1H-1,2,4-triazole-3-methanol*

A mixture of 4-[[[2-[(3-hydroxymethyl-1-methyl-1H-1,2,4-triazol-5-yl)amino]ethyl]thio]methyl]-N,N,N-trimethyl-2-thiophenemethanium iodide (1.12 g) and 2-furanmethanamine (2.25 g) was heated at 140—160° for 2h. The oily residue was washed with diethyl ether (5 × 15 ml) and suspended in water (30 ml) containing anhydrous sodium carbonate (2 g). The suspension was extracted with ethyl acetate (3 × 30 ml), the combined extracts dried and evaporated *in vacuo*. The oily residue was chromatographed (silica/ethyl acetate-methanol, 1:1) to give the *title compound* (0.27 g) as an oil.

T.l.c. ethyl acetate:methanol, 1:1 Rf 0.65.

N.M.R. ($CDCl_3$) 2.60, m, (1H); 3.00, brs, (1H); 3.09, brs, (1H); 3.70, dd, (1H); 3.80, d, (1H); 5.50, s+t, (3H); 6.13, s, (2H); 6.23, s, (2H); 6.36, s, 6.52, s+q, (7H); 6.83, brs, (2H); 7.31, t, (2H).

(b) Prepared in a similar manner was:

5 - [[2 - [[5 - ['[(Phenylmethyl)amino]methyl] - 3 - thienylmethyl]thio]ethyl]amino] - 1 - methyl-1H - 1,2,4 - triazole - 3 - methanol (0.40 g) as an oil from 4 - [[[2 - [(3 - hydroxymethyl - 1 - methyl-1H - 1,2,4 - triazol - 5 - yl)amino]ethyl]thio]methyl] - N,N,N - trimethyl - 2 - thiophenemethanium iodide (1.8 g) and benzenemethanamine (1.0 g) at 120° for 4h followed by evaporation of the mixture with water (3 × 20 ml) *in vacuo* and column chromatography (silica; ethyl acetate-methanol, 3:1)

T.l.c. ethyl acetate-methanol, 1:1 Rf 0.55

N.M.R. ($CDCl_3$) 2.70, m, (5H); 3.03, brs, (1H); 3.13, brs, (1H); 5.49, s, 5.50, t, (3H); 6.10, s, (2H); 6.20, s, (2H); 6.38, s, 6.54, s+q, (7H); 6.90, brs, (2H); 7.32, t, (2H).

(c) Prepared in a similar way was:

5 - [[2 - [[5 - [(Heptylamino)methyl] - 3 - thienylmethyl]thio]ethyl]amino] - 1 - methyl - 1H - 1,2,4-triazole - 3 - methanol (0.3 g) from 4 - [[[2 - [(3 - hydroxymethyl - 1 - methyl - 1*H* - 1,2,4 - triazole - 5-yl)amino]ethyl]thio]methyl] - N,N,N - trimethyl - 2 - thiophenemethanium iodide (1.8 g) and heptanamine (2.15 g) at 120° for 1h and 140° for 4h followed by evaporation of the residue with water (4 x 20 ml) *in vacuo* and column chromatography on the residue (silica/methanol-acetyl acetate, 3:1 then silica/ethanol)

N.M.R. ($CDCl_3$): 3.03, d, (1H); 3.13, d, (1H); 5.36, t, (1H); 5.5, s, (2H); 6.15, m, (2H); 6.38, s, (2H); 6.53, s, (3H); 6.55, q, (2H); 6.75, s, (br), (2H); 7.3 m, (4H); 8.3—9.0, m, (10H); 9.13, t, (3H).

T.l.c. ethanol Rf 0.32

Example 9

*5 - (4 - [[5 - (Dimethylamino)methyl - 4 - methyl - 2 - thienyl]butyl]amino] - 1 - methyl - 1H-1,2,4 - triazole - 3 - methanol*

A mixture of 5 - [4 - [(3 - hydroxymethyl - 1 - methyl - 1H - 1,2,4 - triazol - 5 - yl)amino]butyl - 3-methyl - 2 - thiophenecarboxaldehyde (0.55 g) and dimethylamine in industrial methylated spirits (33% w/w) 8 ml was heated at 50° for 18h. The mixture was cooled and sodium borohydride (0.5 g) was added. The mixture was stirred at room temperature for 2h, acidified with 2N hydrochloric acid and washed with ethyl acetate. The aqueous phase was basified with sodium carbonate solution and extracted with ethyl acetate. The organic extract was evaporated to give an oil which was purified by column chromatography on silica on silica using 2:1 ethyl acetate-methanol as eluant to give the *title compound* as a light brown oil (0.22 g).

N.M.R. ($CDCl_3$: 3.68, s, (1H); 5.47, s, (2H); 5.90; t, (1H); 6.3, br.s, (1H); 6.5—6.7, m, 7H; 7.25, m, (2H); 7.78, s, (6H); 7.90, s, (3H); 8.3, m, (4H).

I.R. ($CHBr_3$): 3585, 3420, 2820, 2770, 1530 $cm^{-1}$.

The ability of the compounds of the invention to inhibit histamine induced gastric acid secretion has been demonstrated in the perfused rat stomach preparation referred to previously; the effectiveness of a compound conveniently being expressed in terms of its $ED_{50}$ (mg/kg) value. The results obtained for a representative number of compounds according to the invention are given below:

| Example No. | $ED_{50}$ |
|---|---|
| 1 (a) | 0.054 |
| 1 (d) | 0.062 |
| 1 (e) | 0.022 |
| 1 (i) | 0.063 |
| 2 (d) | 0.074 |
| 5 (b) | 0.082 |
| 5 (c) | 0.18 |
| 6 (a) | 0.073 |

The compounds of the invention have in general low toxicity at doses at which they effectively inhibit gastric acid secretion. Thus for instance the compounds of Examples 1 (a), 1 (d), 1 (e),1 (i) and 2 (d) produced no toxic effects when administered intravenously to anaesthetised rats at doses in excess of 13 times their $ED_{50}$ values.

## Claims

1. Compounds of the general formula (I)

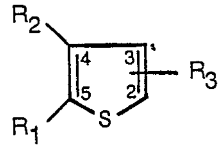

and physiologically acceptable salts and hydrates thereof, in which one of $R_1$ and $R_2$ represents

hydrogen, halogen or a $C_{1-4}$ alkyl group which may be optionally substituted by hydroxy or $C_{1-4}$ alkoxy, and the other represents the group $R_4R_5NAlk$—

in which $R_4$ represents hydrogen, $C_{1-10}$ alkyl, $C_{3-8}$ cycloalkyl, $C_{3-6}$ alkenyl, $C_{3-6}$ alkynyl, $C_{1-6}$ trifluoroalkyl, $C_{1-6}$ alkyl substituted by phenyl, substituted phenyl, hydroxy, $C_{1-6}$ alkoxy, amino, $C_{1-6}$ alkylamino, di ($C_{1-6}$ alkylamino or $C_{3-8}$ cycloalkyl,

or $R_4$ represents $C_{1-4}$ alkyl substituted by a 5 or 6 membered monocyclic heteroaryl ring containing one or more heteroatoms selected from oxygen, nitrogen and sulphur, which heteroaryl ring is unsubstituted or substituted by $C_{1-3}$ alkyl, $C_{1-3}$ alkoxy, hydroxy, amino $C_{1-6}$ alkyl, $C_{1-6}$ alkylamino $C_{1-6}$ alkyl, di $C_{1-6}$ alkylamino $C_{1-6}$ alkyl or halogen, and which heteroaryl ring is linked to the alkyl group through either a carbon or a nitrogen atom,

and $R_5$ represents hydrogen or a $C_{1-4}$ alkyl group,

or $R_4$ and $R_5$ may, together with the nitrogen atom to which they are attached, form a 5 to 10 membered ring which may be saturated or may contain at least one double bond, may be unsubstituted or may be substituted by one or more $C_{1-3}$ alkyl groups or a hydroxy group and/or may contain another heteroatom selected from oxygen and sulphur;

Alk represents a straight or branched alkylene chain of 1 to 6 carbon atoms;

$R_3$, which may be in either the 2- or 3-position, represents the group $—(CH_2)_nX(CH_2)_mNH—Z$ where X represents $—CH_2—$, —O— or —S—;

n represents zero, 1 or 2;

m represents 2, 3 or 4; and

Z represents either the group

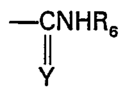

or the group

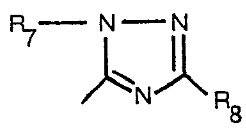

where Y represents S, O, $CHNO_2$ or $NR_9$ where $R_9$ is nitro, cyano, $C_{1-6}$ alkylsulphonyl, phenylsulphonyl or substituted phenyl sulphonyl;

$R_6$ represents hydrogen, $C_{1-6}$ alkyl, $C_{3-6}$ alkenyl, $C_{3-6}$ alkynyl, $C_{1-6}$ alkoxy $C_{1-6}$ alkyl or $C_{1-6}$ alkyl substituted by phenyl or substituted phenyl;

$R_7$ represents hydrogen, $C_{1-6}$ alkyl, $C_{3-6}$ alkenyl, $C_{1-6}$ alkyl substituted by phenyl or substituted phenyl, or $C_{2-6}$ alkyl substituted by hydroxy or $C_{1-6}$ alkoxy; and

$R_8$ represents hydrogen, $C_{1-6}$ alkyl, $C_{3-6}$ alkenyl, $C_{1-6}$ alkyl substituted by phenyl or substituted phenyl, hydroxy $C_{1-6}$ alkyl, acyloxy $C_{1-6}$ alkyl where the acyl portion is benzoyl, substituted benzoyl, $C_{1-6}$ alkanoyl or $C_{2-7}$ alkanoyl substituted by phenyl or substituted phenyl, $C_{1-6}$ alkoxy $C_{1-6}$ alkyl, aryloxy $C_{1-6}$ alkyl in which the aryl group is phenyl or substituted phenyl, aryl $C_{1-6}$ alkyloxy $C_{1-6}$ alkyl in which the aryl group is phenyl or substituted phenyl, amino $C_{1-6}$ alkyl, $C_{1-6}$ alkylamino $C_{1-6}$ alkyl, di $C_{1-6}$ alkylamino $C_{1-6}$ alkyl, hydroxy or $C_{1-6}$ alkoxy, or the group $NR_{10}R_{11}$ where

$R_{10}$ represents hydrogen, $C_{1-6}$ alkyl, $C_{1-6}$ alkyl substituted by hydroxy or $C_{1-3}$ alkoxy, $C_{3-6}$ alkenyl, $C_{1-6}$ alkyl substituted by phenyl or substituted phenyl or $C_{1-4}$ alkyl substituted by a 5 or 6 membered monocyclic heteroaryl ring containing one or more heteroatoms selected from oxygen, nitrogen and sulphur, which heteroaryl ring is unsubstituted or substituted by $C_{1-3}$ alkyl, $C_{1-3}$ alkoxy, hydroxy, amino-$C_{1-6}$ alkyl, $C_{1-6}$ alkylamino $C_{1-6}$ alkyl, di $C_{1-6}$ alkylamino $C_{1-6}$ alkyl or halogen, and which heteroaryl ring is linked to the alkyl group through either a carbon or a nitrogen atom.

and $R_{11}$ represents any of the groups defined for $R_{10}$ or may represent $COR_{12}$ where

$R_{12}$ represents hydrogen, $C_{1-6}$ alkyl, phenyl, substituted phenyl, $C_{1-6}$ alkyl substituted by phenyl or substituted phenyl, $C_{1-6}$ alkoxy, a 5 or 6 membered monocyclic heteroaryl ring containing one or more heteroatoms selected from oxygen, nitrogen and sulphur, which heteroaryl ring is unsubstituted or substituted by $C_{1-3}$ alkyl, $C_{1-3}$ alkoxy, hydroxy, amino-$C_{1-6}$ alkyl, $C_{1-6}$ alkylamino $C_{1-6}$ alkyl, di $C_{1-6}$ alkylamino $C_{1-6}$ alkyl or halogen, or

$C_{1-4}$ alkyl substituted by a 5 or 6 membered monocyclic heteroaryl ring containing one or more heteroatoms selected from oxygen, nitrogen and sulphur, which heterary ring is unsubstituted or substituted by $C_{1-3}$ alkyl, $C_{1-3}$ alkoxy, hydroxy, amino $C_{1-6}$ alkyl, $C_{1-6}$ alkylamino $C_{1-6}$ alkyl, di $C_{1-6}$ alkylamino $C_{1-6}$ alkyl or halogen, and which heteroaryl ring is linked to the alkyl group through either a carbon or a nitrogen atom, or

$R_{11}$ represents the group $SO_2R_{13}$ where $R_{13}$ represents $C_{1-6}$ alkyl, phenyl or substituted phenyl, or $R_{11}$ represents the group

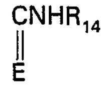

where E represents oxygen or sulphur, and $R_{14}$ represents hydrogen, $C_{1-6}$ alkyl, $C_{3-8}$ cycloalkyl, phenyl, substituted phenyl or $C_{1-6}$ alkyl substituted by phenyl or substituted phenyl, or

$R_{10}$ and $R_{11}$ taken together represent the group $=CR_{15}R_{16}$ where $R_{15}$ represents phenyl, substituted phenyl or a 5 or 6 membered monocyclic heteroaryl ring containing one or more heteroatoms selected from oxygen, nitrogen and sulphur, which heteroaryl ring is unsubstituted or substituted by $C_{1-3}$ alkyl, $C_{1-3}$ alkoxy, hydroxy, amino-$C_{1-6}$ alkyl, $C_{1-6}$ alkylamino $C_{1-6}$ alkyl, di $C_{1-6}$ alkylamino $C_{1-6}$ alkyl or halogen, and $R_{16}$ represents hydrogen or $C_{1-6}$ alkyl,

wherein the terms "substituted phenyl" and "substituted benzoyl" mean phenyl or benzoyl substituted in the phenyl ring by one or more $C_{1-3}$ alkyl or $C_{1-3}$ alkoxy groups or halogen atoms, and

with the provisos that

where $R_2$ represents the group $R_4R_5NAlk$ then $R_3$ is in the 2- position; and

where $R_2$ represents hydrogen then $R_3$ is in the 3- position.

2. Compounds as claimed in Claim 1, characterised in that the groups $R_1$, $R_2$, $R_4$, $R_5$, $R_6$, $R_7$, $R_8$, Y and Alk have the following meanings:—

$R_1$ or $R_2$ (where other than the group $R_4R_5NAlk$):

a hydrogen atom, a bromine atom or a $C_{1-3}$ alkyl group or a $C_{1-3}$ alkoxy $C_{1-3}$ alkyl group or a hydroxy $C_{1-3}$ alkyl group;

or, when $R_1$ or $R_2$ is the group $R_4R_5$ Alk,

$R_4$: $C_{1-10}$ alkyl, $C_{5-7}$ cycloalkyl, $C_{3-6}$ alkenyl, $C_{1-6}$ alkyl substituted by phenyl or substituted phenyl, $C_{1-4}$ alkyl substituted by a trifluoromethyl group, hydroxy $C_{2-4}$ alkyl, $C_{1-3}$ alkoxy $C_{2-4}$ alkyl, di $C_{1-3}$ alkylamino $C_{1-6}$ alkyl, or heteroaralkyl where the heterocyclic portion represents a furyl, thienyl, pyrrolyl, pyridinyl, pyrimidinyl, triazinyl, oxazolyl, triazolyl, or thiazolyl ring and the alkylene portion is a methylene, ethylene or propylene grouping; and

$R_5$: hydrogen or a methyl or ethyl group; or $R_4R_5N$ may represent a 5 to 8 membered ring optionally containing one double bond and/or substituted by one or two $C_{1-3}$ alkyl groups or a hydroxy group and/or containing an oxygen or sulphur atom;

$R_6$: hydrogen, $C_{1-4}$ alkyl or $C_{1-3}$ alkoxy $C_{2-4}$ alkyl;

Y: $CHNO_2$ or $NR_9$ where $R_9$ is nitro, cyano, $C_{1-4}$ alkylsulphonyl, phenyl sulphonyl or substituted phenyl sulphonyl;

$R_7$: hydrogen, $C_{1-4}$ alkyl or hydroxy-$C_{2-4}$ alkyl;

$R_8$: hydrogen, hydroxy, $C_{1-4}$ alkyl, hydroxy $C_{1-4}$ alkyl, $C_{1-3}$ alkoxy $C_{1-4}$ alkyl, phenyl $C_{1-3}$ alkyl, $C_{2-4}$ alkanoyloxy $C_{1-4}$ alkyl, amino $C_{1-4}$ alkyl, amino, $C_{1-4}$ alkylamino or di-$C_{1-4}$ alkylamino, phenyl $C_{1-3}$ alkylamino, or a heteroaryl $C_{1-3}$ alkylamino group where the heteroaryl ring is 5 or 6 membered and contains one heteroatom; or represents the group $NHCOR_{12}$ where $R_{12}$ represents hydrogen, $C_{1-3}$ alkyl, $C_{1-3}$ alkoxy, furyl, pyridyl, thiazolyl, thienyl, or phenyl optionally substituted by a $C_{1-3}$ alkyl or $C_{1-3}$ alkoxy group; or represents the group $NHSO_2R_{13}$ where $R_{13}$ represents $C_{1-3}$ alkyl or phenyl optionally substituted by a $C_{1-3}$ alkyl or $C_{1-3}$ alkoxy group; or represents the group $NHCONHR_{14}$ where $R_{14}$ is $C_{1-3}$ alkyl, $C_{5-7}$ cycloalkyl or phenyl optionally substituted by a $C_{1-3}$ alkyl or $C_{1-3}$ alkoxy group, or represents the group $N{=}CHR_{15}$ where $R_{15}$ is a phenyl or pyridyl group;

Alk is $(CH_2)_p$ where p is 1 to 3.

3. Compounds as claimed in Claim 1 or 2 characterised in that $R_3$ is in the 3- position and $R_1$ is the group $R_4R_5NAlk$.

4. Compounds as claimed in any of Claims 1 to 3, characterised in that Z represents the group

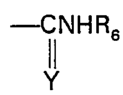

where Y represents the group $CHNO_2$.

5. Compounds as claimed in any of Claims 1 to 3 characterised in that Z represents the group

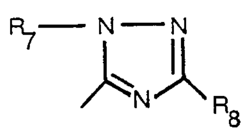

where $R_7$ is hydrogen or methyl, and $R_8$ is amino, hydroxy $C_{1-4}$ alkyl, $C_{1-3}$ alkoxy $C_{1-4}$ alkyl, benzyl, formamido, $C_{2-4}$ alkanoylamino, amino $C_{1-4}$ alkyl, $C_{2-4}$ alkanoyloxy $C_{1-4}$ alkyl, hydroxy, benzoylamino, or phenycarbamoylamino.

6. Compounds as claimed in Claim 5, characterised in that $R_8$ represents amino, hydroxy $C_{1-4}$ alkyl, $C_{2-4}$ alkanoylamino or benzyl.

7. Compounds as claimed in Claim 1, characterised in that they correspond to formula (II)

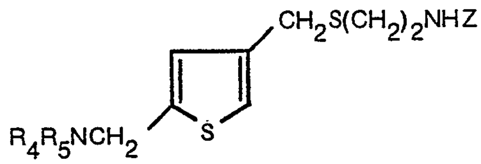

(II)

where $R_4$ and $R_5$ are methyl groups or together with the nitrogen atom to which they are attached form a pyrrolidino, piperidino or hexamethylenimino group, and Z represents either

$$\begin{array}{l}—CNHR_6\\ \;\;\|\\ \;\;CHNO_2\end{array}$$

where $R_6$ is methyl, or the group

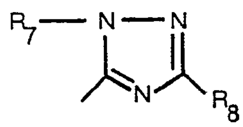

where $R_7$ is hydrogen or methyl, and $R_8$ is amino, hydroxy $C_{1-4}$ alkyl, $C_{1-3}$ alkoxy $C_{1-4}$ alkyl, benzyl, formamido, $C_{2-4}$ alkanoylamino, amino $C_{1-4}$ alkyl, $C_{2-4}$ alkanoyloxy $C_{1-4}$ alkyl, hydroxy, benzoylamino or phenylcarbamoylamino.

8. Compounds as claimed in Claim 1 which are:

N-methyl-N'-[2-[[5-(dimethylaminomethyl)-3-thienylmethyl]thio]ethyl]-2-nitro-1,1-ethenediamine

N-methyl-N'-[2-[[5-(1-pyrrolidinylmethyl)-3-thienylmethyl]thio]ethyl]-2-nitro-1,1-ethenediamine

N-methyl-N'-[2-[[5-(1-piperidinylmethyl)-3-thienylmethyl]thio]ethyl]-2-nitro-1,1-ethenediamine

1-methyl-5-[[2-[[5-(dimethylaminoethyl)-3-thienylmethyl]thio]ethyl]amino]-1H-1,2,4-triazole-3-methanol

5-[[2-[[5-(dimethylaminomethyl)-3-thienyl]methyl]thio]ethyl]amino-3-phenylmethyl-1H-1,2,4-triazole

and their physiologically acceptable salts.

9. Compounds as claimed in Claim 1 which are:

N-[2-[[5-(dimethylaminomethyl)-4-methyl-2-thienylmethyl]thio]ethyl]-N'-methyl-2-nitro-1,1-ethenediamine

1-methyl-$N^5$-[2-[[5-[(dimethylamino)methyl]-4-methyl-2-thienylmethyl]thio]ethyl]-1H-1,2,4-triazole-3,5-diamine

5-[[2-[[[5-(dimethylaminomethyl)-4-methyl]-2-thienylmethyl]thio]ethyl]amino]-1-methyl-1H-1,2,4-triazole-3-methanol

and their physiologically acceptable salts.

10. The compound as claimed in Claim 1 which is:

N-[3-[5-(dimethylaminoethyl)-3-thienylmethoxy]propyl]-N'-methyl-2-nitro-1,1-ethenediamine

and its physiologically acceptable salts.

11. Compounds of formula (I) as defined in Claim 1 characterised in that one of $R_1$ and $R_2$ represents hydrogen and the other represents $R_4R_5NAlk$ and $R_3$ represents

$$\begin{array}{l}—(CH_2)_nX(CH_2)_mNHCNHR_6\\ \qquad\qquad\qquad\quad\;\;\|\\ \qquad\qquad\qquad\quad\;\;Y\end{array}$$

except that Y does not represent $NR_9$ where $R_9$ is $C_{1-6}$ alkylsulphonyl, phenylsulphonyl or substituted phenylsulphonyl.

12. Compounds of formula (I) as defined in Claim 1, characterised in that one of $R_1$ and $R_2$ represents hydrogen and the other represents $R_4R_5NAlk$ and $R_3$ represents

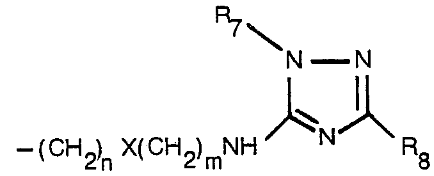

where $R_8$ represents hydrogen, $C_{1-6}$ alkyl, $C_{3-6}$ alkenyl, $C_{1-6}$ alkyl substituted by phenyl or substituted phenyl, hydroxy $C_{1-6}$ alkyl, $C_{1-6}$ alkoxy $C_{1-6}$ alkyl, hydroxy or $C_{1-6}$ alkoxy or $R_8$ represents the group $NR_{10}R_{11}$ where

$R_{10}$ represents hydrogen, $C_{1-6}$ alkyl, hydroxy $C_{1-6}$ alkyl, $C_{3-6}$ alkenyl or $C_{1-6}$ alkyl substituted by phenyl or substituted phenyl and $R_{11}$ represents hydrogen, $C_{1-6}$ alkyl, hydroxy $C_{1-6}$ alkyl, or the group $COR_{12}$ where

$R_{12}$ represents hydrogen, $C_{1-6}$ alkyl, phenyl, substituted phenyl, $C_{1-6}$ alkoxy or $C_{1-6}$ alkyl substituted by phenyl or substituted phenyl, or

$R_{11}$ represents the group $SO_2R_{13}$ where $R_{13}$ represents $C_{1-6}$ alkyl, phenyl or substituted phenyl, or

$R_{10}$ and $R_{11}$ taken together represent the group $=CR_{15}R_{16}$ where $R_{15}$ represents phenyl or substituted phenyl and $R_{16}$ represents hydrogen or $C_{1-6}$ alkyl.

13. A process for the production of compounds of formula (I) as defined in Claim 1, characterised in that

(a) for the production of compounds in which Z represents the group

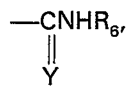

an amine of formula $R_{17}NH_2$ (III) is reacted with a compound of general formula (IV)

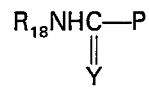

in which one of the groups $R_{17}$ and $R_{18}$ represents the group

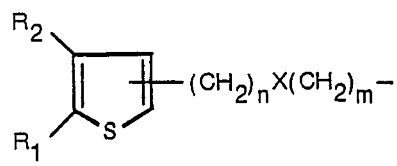

and the other represents the group $R_6$, and P is a leaving group;

(b) for the production of compounds in which Z represents the group

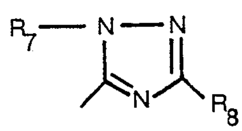

where $R_8$ is other than a $C_{1-6}$ alkoxy or acyloxy $C_{1-6}$ alkyl group, or the group $—N=CR_{15}R_{16}$, a compound of formula (VI)

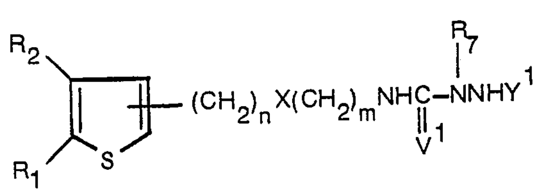

(VI)

in which $V^1$ is

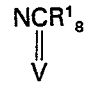

or NCN and $Y^1$ is hydrogen where V is oxygen or sulphur and $R^1_8$ is a group as defined for $R_8$ or is a group convertible thereto under the conditions of the reaction or represents halogen or $C_{1-6}$ alkoxy, or $V^1$ is NH and $Y^1$ is

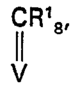

is cyclised;

(c) for the production of compounds in which Alk is $CH_2$ and Z represents the group

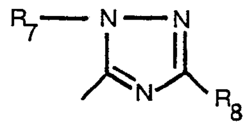

in which $R_8$ is other than the group $—N{=}CR_{15}R_{16}$, an intermediate formed by reacting a compound of formula (VIII)

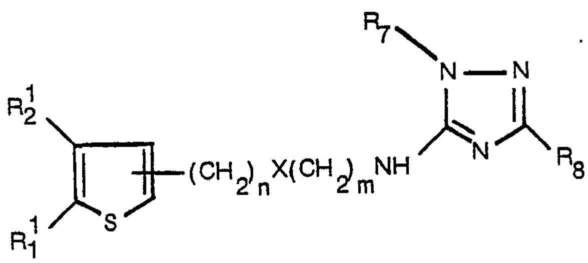

(VIII)

in which one of $R^1_1$ and $R^1_2$ represents hydrogen, halogen or a $C_{1-4}$ alkyl group which may be optionally substituted by hydroxy or $C_{1-4}$ alkoxy and the other represents the group —CHO, with a reagent $R_4R_5NH$, is reduced:

(d) for the production of compounds in which $R_8$ is other than $—N{=}CR_{15}R_{16}$, a compound of formula (I) in which $R_4$ is hydrogen is reacted with an aldehyde $R^1_4$—CHO under reducing conditions, the group $R^1_4$ having a meaning such that the resulting group $R^1_4CH_2$ represents a group $R_4$ as defined;

(e) for the production of compounds in which Z represents the group

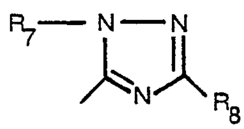

in which $R_8$ is an acyloxy $C_{1-6}$ alkyl group, the corresponding hydroxy $C_{1-6}$ alkyl compound is treated with an appropriate acid at elevated temperature;

(f) for the production of compounds in which Z represents the group

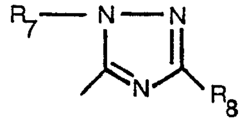

in which the group $R_8$ represents the group $—N{=}CR_{15}R_{16}$, a compound of formula (I) in which $R_8$ is an $NH_2$ group is reacted with an aldehyde or ketone $R_{15}R_{16}CO$;

(g) for the production of compounds in which Z represents the group

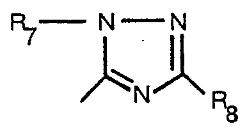

in which $R_8$ is the group $NR_{10}R_{11}$ where $R_{11}$ is $—COR_{12}$, $—SO_2R_{13}$ or $—C(=E)NHR_{14}$, a compound of formula (I) in which $R_8$ is the group $—NHR_{10}$ and $R_1$, $R_2$, $R_7$ and $R_{10}$ are as defined in formula

(I) or are groups readily convertible thereto is treated with a reagent capable of replacing the hydrogen atom in the group $NHR_{10}$ by the group $R_{11}$;

(h) treating a compound of formula (I) in which the group $R_4R_5N$ is replaced by a quaternary ammonium group with an amine $R_4R_5NH$;

and where the compound of formula (I) is produced in the form of a free base, optionally converting the free base into a salt.

14. A pharmaceutical composition comprising a compound as claimed in any of Claims 1 to 12, together with at least one pharmaceutically acceptable carrier or diluent, and optionally one or more further active ingredients.

**Revendications**

1. Composés de formule générale (I)

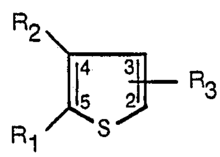

et leurs sels et hydrates physiologiquement acceptables, où l'un des radicaux $R_1$ et $R_2$ représente un hydrogène, un halogène ou un groupe alcoyle en $C_1$ à $C_4$ qui peut être éventuellement substitué par un hydroxy ou un alcoxy en $C_1$ à $C_4$, et l'autre représente le groupe $R_4R_5NAlc—$

où $R_4$ représente un hydrogène, un alcoyle en $C_1$ à $C_{10}$, un cycloalcoyle en $C_3$ à $C_8$, un alcényle en $C_3$ à $C_6$, un alcynyle en $C_3$ à $C_6$, un trifluoroalcoyle en $C_1$ à $C_6$, un alcoyle en $C_1$ à $C_6$ substitué par un phényle, un phényle substitué, un hydroxy, un alcoxy en $C_1$ à $C_6$, un amino, un alcoyle en $C_1$ à $C_6$-amino, un di-alcoyle en $C_1$ à $C_6$-amino ou un cycloalcoyle en $C_3$ à $C_8$,

ou $R_4$ représente un alcoyle en $C_1$ à $C_4$ substitué par un noyau hétéroaryle monocyclique à 5 ou 6 chaînons contenant un ou plusieurs hétéroatomes choisis entre l'oxygène, l'azote et le soufre, lequel noyau hétéroaryle est non substitué ou substitué par un alcoyle en $C_1$ à $C_3$, un alcoxy en $C_1$ à $C_3$, un hydroxy, un amino-alcoyle en $C_1$ à $C_6$, un alcoyle en $C_1$ à $C_6$-amino-alcoyle en $C_1$ à $C_6$, un di-alcoyle en $C_1$ à $C_6$-amino-alcoyle en $C_1$ à $C_6$ ou un halogène, et lequel noyau hétéroaryle est lié au groupe aryle par une liaison carbone ou azote,

et $R_5$ représente un hydrogène ou un groupe alcoyle en $C_1$ à $C_4$,

ou $R_4$ et $R_5$ peuvent, avec l'atome d'azote auquel ils sont attachés, former un noyau à 5 à 10 chaînons qui peut être saturé ou peut contenir au moins une double liaison, peut être non substitué ou peut être substitué par un ou plusieurs groupes alcoyle en $C_1$ à $C_3$ ou un groupe hydroxy et/ou peuvent contenir un autre hétéroatome choisi entre l'oxygène et le soufre,

Alc représente une chaîne alcoylène droite ou ramifiée en $C_1$ à $C_6$;

$R_3$, qui peut être en position 2 ou en position 3, représente le groupe $—(CH_2)_nX(CH_2)_mNH—Z$ où X représente $—CH_2—$, $—O—$ ou $—S—$;

n représenté zéro, 1 ou 2;

m représente 2, 3 ou 4; et

Z représente soit le groupe

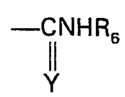

soit le groupe

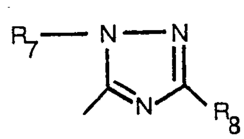

où Y représente S, O, $CHNO_2$ ou $NR_9$ où $R_9$ est un nitro, un cyano, un alcoyle en $C_1$ à $C_6$-sulfonyle, un phénylsulfonyle ou un phénylsulfonyle substitué;

$R_6$ représente un hydrogène, un alcoyle en $C_1$ à $C_6$, un alcényle en $C_3$ à $C_6$, un alcynyle en $C_3$ à $C_6$, un alcoxy en $C_1$ à $C_6$-alcoyle en $C_1$ à $C_6$ ou un alcoyle en $C_1$ à $C_6$ substitué par un phényle ou un phényle substitué;

$R_7$ représente un hydrogène, un alcoyle en $C_1$ à $C_6$, un alcényle en $C_3$ à $C_6$, un alcoyle en $C_1$ à $C_6$ substitué par un phényle ou un phényle substitué, ou un alcoyle en $C_2$ à $C_6$ substitué par un hydroxy ou un alcoxy en $C_1$ à $C_6$; et

$R_8$ représente un hydrogène, un alcoyle en $C_1$ à $C_6$, un alcényle en $C_3$ à $C_6$, un alcoyle en $C_1$ à $C_6$ substitué par un phényle ou un phényle substitué, un hydroxy-alcoyle en $C_1$ à $C_6$, un acyloxy-alcoyle en $C_1$ à $C_6$ où la fraction acyle est un benzoyle, un benzoyle substitué, un alcanoyle en $C_1$ à $C_6$ ou un alcanoyle en $C_1$ à $C_6$ substitué par un phényle ou un phényle substitué, un alcoxy en $C_1$ à $C_6$-alcoyle en $C_1$ à $C_6$, un aryloxy-alcoyle en $C_1$ à $C_6$ où le groupe aryle est un phényle ou un phényle substitué, un aryl-alcoyloxy en $C_1$ à $C_6$-alcoyle en $C_1$ à $C_6$ où le groupe aryle est un phényle ou un phényle substitué, un amino-alcoyle en $C_1$ à $C_6$, un alcoyle en $C_1$ à $C_6$-amino-alcoyle en $C_1$ à $C_6$, un di-alcoyle en $C_1$ à $C_6$-amino-alcoyle en $C_1$ à $C_6$, un hydroxy ou un alcoxy en $C_1$ à $C_6$, ou le groupe $NR_{10}R_{11}$ où

$R_{10}$ représente un hydrogène, un alcoyle en $C_1$ à $C_6$, un alcoyle en $C_1$ à $C_6$ substitué par un hydroxy ou un alcoxy en $C_1$ à $C_3$, un alcényle en $C_3$ à $C_6$, un alcoyle en $C_1$ à $C_6$ substitué par un phényle ou un phényle substitué ou un alcoyle en $C_1$ à $C_4$ substitué par un noyau hétéroaryle monocyclique à 5 ou 6 chaînons contenant un ou plusieurs hétéroatomes choisis entre l'oxygène, l'azote et le soufre, lequel noyau hétéroaryle est non substitué ou substitué par un alcoyle en $C_1$ à $C_3$, un alcoxy en $C_1$ à $C_3$, un hydroxy, un amino-alcoyle en $C_1$ à $C_6$, un alcoyle en $C_1$ à $C_6$-amino-alcoyle en $C_1$ à $C_6$, un di-alcoyle en $C_1$ à $C_6$-amino-alcoyle en $C_1$ à $C_6$ ou un halogène, et lequel noyau hétéroaryle est lié au groupe alcoyle par un atome de carbone ou un atome d'azote,

et $R_{11}$ représente l'un quelconque des groupes définis pour $R_{10}$ ou peut représenter $COR_{12}$ où

$R_{12}$ représente un hydrogène, un alcoyle en $C_1$ à $C_6$, un phényle, un phényle substitué, un alcoyle en $C_1$ à $C_6$ substitué par un phényle ou un phényle substitué, un alcoxy en $C_1$ à $C_6$, un noyau hétéroaryle monocyclique à 5 ou 6 chaînons contenant un ou plusieurs hétéroatomes choisis entre l'oxygène, l'azote et le soufre, lequel noyau hétéroaryle est non substitué ou substitué par un alcoyle en $C_1$ à $C_3$, un alcoxy en $C_1$ à $C_3$, un hydroxy, un amino-alcoyle en $C_1$ à $C_6$, un alcoyle en $C_1$ à $C_6$-amino-alcoyle en $C_1$ à $C_6$, un dialcoyle en $C_1$ à $C_6$-amino-alcoyle en $C_1$ à $C_6$ ou un halogène, ou

un alcoyle en $C_1$ à $C_4$ substitué par un noyau hétéroaryle monocyclique à 5 ou 6 chaînons contenant un ou plusieurs hétéroatomes choisis entre l'oxygène, l'azote et le soufre, lequel noyau hétéroaryle est non substitué ou substitué par un alcoyle en $C_1$ à $C_3$, un alcoxy en $C_1$ à $C_3$, un hydroxy, un amino-alcoyle en $C_1$ à $C_6$, un alcoyle en $C_1$ à $C_6$-amino-alcoyle en $C_1$ à $C_6$, un di-alcoyle en $C_1$ à $C_6$-amino-alcoyle en $C_1$ à $C_6$ ou un halogène, et lequel noyau hétéroaryle est lié au groupe alcoyle par un atome de carbone ou d'azote, ou

$R_{11}$ représente le groupe $SO_2R_{13}$ où $R_{13}$ représente un alcoyle en $C_1$ à $C_6$, un phényle ou un phényle substitué, ou

$R_{11}$ représente le groupe

$$\underset{\underset{\displaystyle E}{\|}}{C}NHR_{14}$$

où E représente un oxygène ou un soufre, et $R_{14}$ représente un hydrogène, un alcoyle en $C_1$ à $C_6$, un cycloalcoyle en $C_3$ à $C_8$, un phényle, un phényle substitué ou un alcoyle en $C_1$ à $C_6$ substitué par un phényle ou un phényle substitué, ou

$R_{10}$ et $R_{11}$ pris ensemble représentent le groupe $=CR_{15}R_{16}$ où $R_{15}$ représente un phényle, un phényle substitué ou un noyau hétéroaryle monocyclique à 5 ou 6 chaînons contenant un ou plusieurs hétéroatomes choisis entre l'oxygène, l'azote et le soufre, lequel noyau hétéroaryle est non substitué ou substitué par un alcoyle en $C_1$ à $C_3$, un alcoxy en $C_1$ à $C_3$, un hydroxy, un amino-alcoyle en $C_1$ à $C_6$, un alcoyle en $C_1$ à $C_6$-amino-alcoyle en $C_1$ à $C_6$, un d-alcoyle en $C_1$ à $C_6$-amino-alcoyle en $C_1$ à $C_6$ ou un halogène, et $R_{16}$ représente un hydrogène ou un alcoyle en $C_1$ à $C_6$, et où les termes "phényle substitué" et "benzoyle substitué" désignent un phényle ou un benzyle substitué par un ou plusieurs groupes alcoyle en $C_1$ à $C_3$ ou alcoxy en $C_1$ à $C_3$ ou des halogènes,

avec les précisions que

lorsque $R_2$ représente le groupe $R_4R_5NAlc$, $R_3$ est en position 2; et

lorsque $R_2$ représente un hydrogène, $R_3$ est en position 3.

2. Composés selon la revendication 1, caractérisés en ce que les groupes $R_1$, $R_2$, $R_4$, $R_5$, $R_6$, $R_7$, $R_8$. Y et Alc ont les significations suivantes:

$R_1$ ou $R_2$ (lorsqu'ils sont différents du groupe $R_4R_5NAlc$):

un atome d'hydrogène, un atome de brome ou un groupe alcoyle en $C_1$ à $C_3$ ou un groupe alcoxy en $C_1$ à $C_3$-alcoyle en $C_1$ à $C_3$ ou un groupe hydroxy-alcoyle en $C_1$ à $C_3$;

ou, lorsque $R_1$ ou $R_2$ est le groupe $R_4R_5Alc$,

$R_4$: alcoyle en $C_1$ à $C_{10}$, cycloalcoyle en $C_5$ à $C_7$, alcényle en $C_3$ à $C_6$, alcoyle en $C_1$ à $C_6$ substitué par un phényle ou un phényle substitué, alcoyle en $C_1$ à $C_4$ substitué par un groupe trifluorométhyle, hydroxy-alcoyle en $C_2$ à $C_4$, alcoxy en $C_1$ à $C_3$-alcoyle en $C_2$ à $C_4$, di-alcoyle en $C_1$ à $C_3$-amino-alcoyle en $C_1$ à $C_6$, ou hétéroaralcoyle où la fraction hétérocyclique représente un noyau furyle, thiényle, pyrrolyle, pyridinyle, pyrimidinyle, triazinyle, oxazolyle, triazolyle ou thiazolyle et la fraction alcoylène est un groupement méthylène, éthylène ou propylène; et

$R_5$: hydrogène ou un groupe méthyle ou éthyle; ou $R_4R_5N$ peut représenter un noyau à 5 à 8 chaînons contenant éventuellement un double liaison et/ou substitué par 1 ou 2 groupes alcoyle en $C_1$ à $C_3$ ou un groupe hydroxy et/ou contenant un atome d'oxygène ou de soufre;

$R_6$: hydrogène, alcoyle en $C_1$ à $C_4$ ou alcoxy, en $C_1$ à $C_3$-alcoyle en $C_2$ à $C_4$;

Y: $CHNO_2$ ou $NR_9$ où $R_9$ est un nitro, un cyano, un alcoyle en $C_1$ à $C_4$-sulfonyle, un phénylsulfonyle ou un phénylsulfonyle substitué;

$R_7$: hydrogène, alcoyle en $C_1$ à $C_4$ ou hydroxyalcoyle en $C_2$ à $C_4$;

$R_8$: hydrogène, hydroxy, alcoyle en $C_1$ à $C_4$, hydroxy-alcoyle en $C_1$ à $C_4$, alcoxy en $C_1$ à $C_3$-alcoyle en $C_1$ à $C_4$, phényl-alcoyle en $C_1$ à $C_3$, alcanoyloxy en $C_2$ à $C_4$-alcoyle en $C_1$ à $C_4$, amino-alcoyle en $C_1$ à $C_4$, amino, alcoyle en $C_1$ à $C_4$-amino ou dialcoyle en $C_1$ à $C_4$-amino, phényl-alcoyle en $C_1$ à $C_3$-amino, ou un groupe hétéroaryl-alcoyle en $C_1$ à $C_3$-amino où le noyau hétéroaryle est à 5 ou 6 chaînons et contient un hétéroatome; ou représente le groupe $NHCOR_{12}$ où $R_{12}$ représente un hydrogène, un alcoyle en $C_1$ à $C_3$, un alcoxy en $C_1$ à $C_3$, un furyle, un pyridyle, un thiazolyle, un thiényle, ou un phényle éventuellement substitué par un groupe alcoyle en $C_1$ à $C_3$ ou alcoxy en $C_1$ à $C_3$; ou représente le groupe $NHSO_2R_{13}$ où $R_{13}$ représente un alcoyle en $C_1$ à $C_3$ ou un phényle éventuellement substitué par un groupe alcoyle en $C_1$ à $C_3$ ou alcoxy en $C_1$ à $C_3$; ou représente le groupe $NHCONHR_{14}$ où $R_{14}$ est un alcoyle en $C_1$ à $C_3$, un cycloalcoyle en $C_5$ à $C_7$ ou un phényle éventuellement substitué par un groupe alcoyle en $C_1$ à $C_3$ ou alcoxy en $C_1$ à $C_3$, ou représente le groupe $N{=}CHR_{15}$ où $R_{15}$ est un groupe phényle ou pyridyle;

Alc représente $(CH_2)_p$ où p vaut de 1 à 3.

3. Composés selon l'une des revendications 1 ou 2 caractérisés en ce que $R_3$ est en position 3 et $R_1$ est le groupe $R_4R_5NAlc$.

4. Composés selon l'une quelconque des revendications 1 à 3, caractérisés en ce que Z représente le groupe

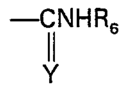

où Y représente le groupe $CHNO_2$.

5. Composés selon l'une quelconque des revendications 1 à 3 caractérisés en ce que Z représente le groupe

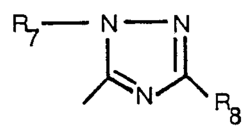

où $R_7$ est un hydrogène ou un méthyle, et $R_8$ est un amino, un hydroxy-alcoyle en $C_1$ à $C_4$, un alcoxy en $C_1$ à $C_3$-alcoyle en $C_1$ à $C_4$, un benzyle, un formamido, un alcanoyl en $C_2$ à $C_4$-amino, un amino-alcoyle en $C_1$ à $C_4$, un alcanoyloxy en $C_2$ à $C_4$-alcoyle en $C_1$ à $C_4$, un hydroxy, un benzoylamino, ou un phénylcarbamoylamino.

6. Composés selon la revendication 5, caractérisés en ce que $R_8$ représente un amino, un hydroxyalcoyle en $C_1$ à $C_4$, un alcanoyle en $C_2$ à $C_4$-amino ou un benzyle.

7. Composés selon la revendication 1, caractérisés en ce qu'ils correspondent à la formule (II)

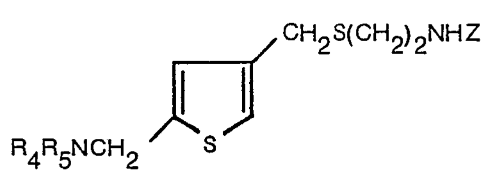

(II)

où $R_4$ et $R_5$ sont des groupes méthyle ou forment avec l'atome d'azote auquel ils sont attachés un groupe pyrrolidino, pipéridino ou hexaméthylèneimino, et Z représente soit

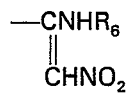

où $R_6$ est un méthyle, soit le groupe

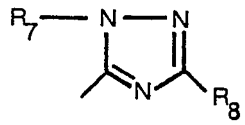

où $R_7$ est un hydrogène ou un méthyle, et $R_8$ est un amino, un hydroxy-alcoyle en $C_1$ à $C_4$, un alcoxy en $C_1$ à $C_3$-alcoyle en $C_1$ à $C_4$, un benzyle, un formamido, un alcanoyle en $C_2$ à $C_4$-amino, un amino-alcoyle en $C_1$ à $C_4$, un alcanoyloxy en $C_2$ à $C_4$-alcoyle en $C_1$ à $C_4$, un hydroxy, un benzoylamino ou un phénylcarbamoylamino.

8. Composés selon la revendication 1 qui sont les suivants:

N-méthyl-N'-[2-[[5-(diméthylaminométhyl)-3-thiénylméthyl]thio]éthyl]-2-nitro-1,1-éthènediamine,

N-méthyl-N'-[2-[[5-(1-pyrrolidinylméthyl)-3-thiénylméthyl]thio]éthyl]-2-nitro-1,1-éthènediamine,

N-méthyl-N'-[2-[[5-(1-pipéridinylméthyl)-3-thiénylméthyl]thio]éthyl]-2-nitro-1,1-éthènediamine,

1-méthyl-5-[[2-[[5-(diméthylaminoéthyl)-3-thiénylméthyl]thio]éthyl]amino]-1H-1,2,4-triazole-3-méthanol,

5-[[2-[[5-(diméthylaminométhyl)-3-thiényl]méthyl]thio]éthyl]amino-3-phénylméthyl-1H-1,2,4-triazole,

et leurs sels physiologiquement acceptables.

9. Composés selon la revendication 1 qui sont les suivants:

N-[2-[[5-(diméthylaminométhyl)-4-méthyl-2-thiénylméthyl]thio]éthyl]-N'-méthyl-2-nitro-1,1-éthènediamine,

1-méthyl-$N^5$-[2-[[5-[(diméthylamino)méthyl]-4-méthyl-2-thiénylméthyl]thio]éthyl]-1H-1,2,4-triazole-3,5-diamine,

5-[[2-[[[5-(diméthylaminométhyl)-4-méthyl]-2-thiénylméthyl]thio]éthyl]amino]-1-méthyl-1H-1,2,4-triazole-3-méthanol,

et leurs sels physiologiquement acceptables.

10. Composé selon la revendication 1 qui est la N-[3-[5-(diméthylaminoéthyl)-3-thiénylméthoxy]propyl]-N'-méthyl-2-nitro-1,1-éthènediamine et ses sels physiologiquement acceptables.

11. Composés de formule (I) selon la revendication 1 caractérisés en ce que l'un des radicaux $R_1$ et $R_2$ représente un hydrogène et l'autre représente $R_4R_5NAlc$ et $R_3$ représente

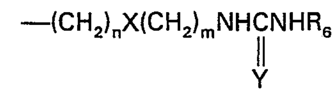

sauf que Y ne représente pas $NR_9$ lorsque $R_9$ est un alcoyle en $C_1$ à $C_6$-sulfonyle, un phénylsulfonyle ou un phénylsulfonyle substitué.

12. Composés de formule (I) définie dans la revendication 1, caractérisés en ce que l'un des radicaux $R_1$ et $R_2$ représente un hydrogène et l'autre représente $R_4R_5NAlc$ et $R_3$ représente

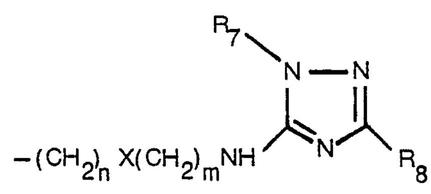

où $R_8$ représente un hydrogène, un alcoyle en $C_1$ à $C_6$, un alcényle en $C_3$ à $C_6$, un alcoyle en $C_1$ à $C_6$ substitué par un phényle ou un phényle substitué, un hydroxy-alcoyle en $C_1$ à $C_6$, un alcoxy en $C_1$ à $C_6$-alcoyle en $C_1$ à $C_6$, un hydroxy ou un alcoxy en $C_1$ à $C_6$ ou $R_8$ représente le groupe $NR_{10}R_{11}$ où

$R_{10}$ représente un hydrogène, un alcoyle en $C_1$ à $C_6$, un hydroxy-alcoyle en $C_1$ à $C_6$, un alcényle en $C_3$ à $C_6$ ou un alcoyle en $C_1$ à $C_6$ substitué par un phényle ou un phényle substitué et $R_{11}$ représente un hydrogène, un alcoyle en $C_1$ à $C_6$, un hydroxy-alcoyle en $C_1$ à $C_6$, ou le groupe $COR_{12}$ où

$R_{12}$ représente un hydrogène, un alcoyle en $C_1$ à $C_6$, un phényle, un phényle substitué, un alcoxy en $C_1$ à $C_6$ ou un alcoyle en $C_1$ à $C_6$ substitué par un phényle ou un phényle substitué, ou

$R_{11}$ représente le groupe $SO_2R_{13}$ où $R_{13}$ représente un alcoyle en $C_1$ à $C_6$, un phényle ou un phényle substitué, ou

$R_{10}$ et $R_{11}$ pris ensemble représentent le groupe $=CR_{15}R_{16}$ où $R_{15}$ représente un phényle ou un phényle substitué et $R_{16}$ représente un hydrogène ou un alcoyle en $C_1$ à $C_6$.

13. Procédé de préparation des composés de formule (I) selon la revendication 1, caractérisé en ce que

(a) pour la préparation des composés où Z représente le groupe

$$-\underset{\underset{Y}{\|}}{C}NHR_6,$$

on fait réagir une amine de formule $R_{17}NH_2$ (III) avec un composé de formule générale (IV)

$$R_{18}NH\underset{\underset{Y}{\|}}{C}-P$$

où l'un des groupes $R_{17}$ et $R_{18}$ représente le groupe

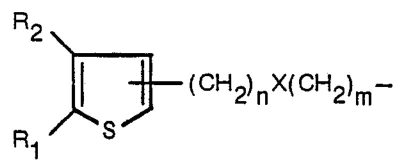

et l'autre représente le groupe $R_6$, et P est un groupe sortant;

(b) pour la préparation des composés où Z représente le groupe

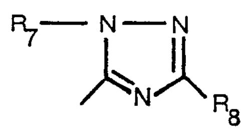

où $R_8$ est différent d'un groupe alcoxy en $C_1$ à $C_6$ ou acyloxy-alcoyle en $C_1$ à $C_6$, ou le groupe $-N=CR_{15}R_{16}$, on cyclise un composé de formule (VI)

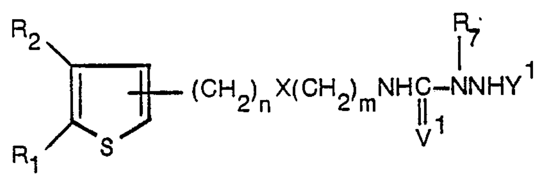

(VI)

où $V^1$ représente

$$\underset{\underset{V}{\|}}{NC}R^1_8$$

ou NCN et $Y^1$ est un hydrogène où V est un oxygène ou un soufre et $R^1_8$ est un groupe tel que défini pour $R_8$ ou est un groupe transformable en un tel groupe dans les conditions de la réaction ou représente un halogene, ou un alcoxy en $C_1$ à $C_6$, ou $V^1$ représente NH et $Y^1$ représente

$$\underset{\underset{V}{\|}}{C}R^1_8;$$

(c) pour la préparation des composés où Alc est $CH_2$ et Z représente le groupe

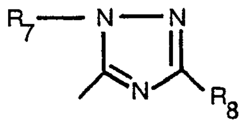

où $R_8$ est différent du groupe —$N{=}CR_{15}R_{16}$, on réduit un intermédiaire formé en faisant réagir un composé de formule (VIII)

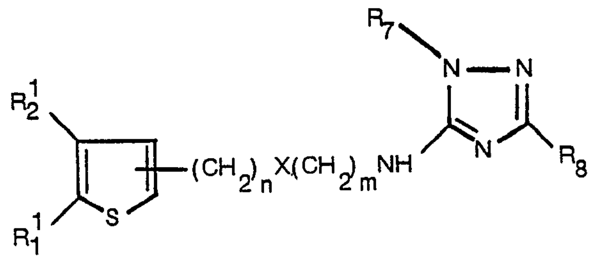

(VIII)

où l'un des radicaux $R^1_1$ et $R^1_2$ représente un hydrogène, un halogène, ou un groupe alcoyle en $C_1$ à $C_4$ qui peut être éventuellement substitué par un hydroxy ou un alcoxy en $C_1$ à $C_4$ et l'autre représente le groupe —CHO, avec un réactif $R_4R_5NH$;

(d) pour la préparation des composés où $R_8$ est différent de —$N{=}CR_{15}R_{16}$, on fait réagir un composé de formule (I) où $R_4$ est un hydrogène avec un aldéhyde $R_4$—CHO dans des conditions réductrices, le groupe $R^1_4$ ayant une signification telle que le groupe $R^1_4CH_2$ obtenu représente un groupe $R_4$ tel que défini;

(e) pour la préparation des composés où Z représente le groupe

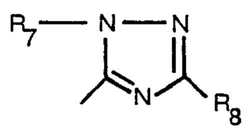

où $R_8$ est un groupe acyloxy-alcoyle en $C_1$ à $C_6$, on traite le composé hydroxy-alcoyle en $C_1$ à $C_6$ correspondant avec un acide approprié à une température élevée;

(f) pour la préparation des composés où Z représente le groupe

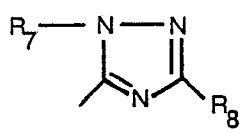

où le groupe $R_8$ représente le groupe —$N{=}CR_{15}R_{16}$, on fait réagir un composé de formule (I) où $R_8$ est un groupe $NH_2$ avec un aldéhyde ou une cétone $R_{15}R_{16}CO$;

(g) pour la préparation des composés où Z représente le groupe

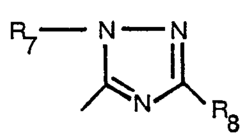

où $R_8$ est le groupe $NR_{10}R_{11}$ où $R_{11}$ représente —$COR_{12}$, —$SO_2R_{13}$ ou —$C({=}E)NHR_{14}$, on traite un composé de formule (I) où $R_8$ est le groupe —$NHR_{10}$ et $R_1$, $R_2$, $R_7$ et $R_{10}$ sont tels que définis dans la formule (I) ou sont des groupes facilement transformables en de tels groupes, avec un réactif capable de remplacer l'atome d'hydrogène dans le groupe $NHR_{10}$ par le groupe $R_{11}$;

(h) on traite un composé de formule (I) où le groupe $R_4R_5N$ est remplacé par un groupe ammonium quaternaire avec une amine $R_4R_5NH$;

et où le composé de formule (I) est produit sous la forme d'une base libre, le cas échéant en transformant la base libre en un sel.

14. Composition pharmaceutique comprenant un composé selon l'une quelconque des revendications 1 à 12, avec au moins un support ou diluant pharmaceutiquement acceptable, et le cas échéant un ou plusieurs autres ingrédients actifs.

**Patentansprüche**

1. Verbindungen der allgemeinen Formel (I):

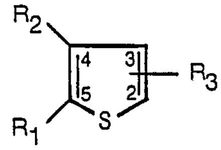

und ihre physiologisch annehmbaren Salze und Hydrate davon, worin einer der Substituenten $R_1$ und $R_2$ für Wasserstoff, Halogen oder eine $C_{1-4}$-Alkylgruppe, die gegebenenfalls durch Hydroxy oder $C_{1-4}$-Alkoxy substituiert sein kann, steht und der andere für die Gruppe $R_4R_5NAlk$ steht, worin $R_4$ für Wasserstoff, $C_{1-10}$-Alkyl, $C_{3-8}$-Cycloalkyl, $C_{3-6}$-Alkenyl, $C_{3-6}$-Alkinyl, $C_{1-6}$-Trifluoralkyl, $C_{1-6}$-Alkyl, substituiert durch Phenyl, substituiertes Phenyl, Hydroxy, $C_{1-6}$-Alkoxy, Amino, $C_{1-6}$-Alkylamino, Di-$C_{1-6}$-alkylamino oder $C_{3-8}$-Cycloalkyl, steht, oder $R_4$ für $C_{1-4}$-Alkyl, substituiert durch einen 5- oder 6-gliedrigen monocyclischen Heteroarylring, enthaltend ein oder mehrere Heteroatome, ausgewählt aus Sauerstoff, Stickstoff und Schwefel, wobei der Heteroarylring unsubstituiert ist oder durch $C_{1-3}$-Alkyl, $C_{1-3}$-Alkoxy, Hydroxy, Amino-$C_{1-6}$-Alkyl, $C_{1-6}$-Alkylamino-$C_{1-6}$-alkyl, Di-$C_{1-6}$-alkylamino-$C_{1-6}$-alkyl oder Halogen substituiert ist, und wobei der Heteroarylring mit der Alkylgruppe entweder durch ein Kohlenstoff- oder ein Stickstoffatom verbunden ist, steht, und $R_5$ für Wasserstoff oder eine $C_{1-4}$-Alkylgruppe steht, oder $R_4$ und $R_5$ zusammen mit dem Stickstoffatom, an das sie angefügt sind, einen 5- bis 10-gliedrigen Ring bilden können, der gesättigt sein kann oder der mindestens eine Doppelbindung enthalten kann, der unsubstituiert sein kann oder der durch eine oder mehrere $C_{1-3}$-Alkylgruppen oder eine Hydroxygruppe substituiert sein kann und/oder der ein weiteres Heteroatom, ausgewählt aus Sauerstoff und Schwefel, enthalten kann, Alk für eine gerade oder verzweigte Alkylenkette mit 1 bis 6 Kohlenstoffatomen steht, $R_3$, das entweder in 2- oder 3-Stellung sein kann, die Gruppe $-(CH_2)_nX(CH_2)_mNH-Z$ bedeutet, worin X für $-CH_2-$, $-O-$ oder $-S-$ steht, n den Wert 0, 1 oder 2 hat, m den Wert 2, 3 oder 4 hat und Z entweder für die Gruppe

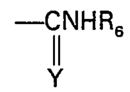

oder die Gruppe

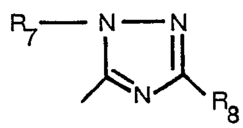

steht, wobei Y für S, O, $CHNO_2$ oder $NR_9$, wobei $R_9$ die Bedeutung Nitro, Cyano, $C_{1-6}$-Alkylsulfonyl, Phenylsulfonyl oder substituiertes Phenylsulfonyl hat, steht, $R_6$ für Wasserstoff, $C_{1-6}$-Alkyl, $C_{3-6}$-Alkenyl, $C_{3-6}$-Alkinyl, $C_{1-6}$-Alkoxy-$C_{1-6}$-alkyl oder $C_{1-6}$-Alkyl, substituiert durch Phenyl oder substituiertes Phenyl, steht, $R_7$ für Wasserstoff, $C_{1-6}$-Alkyl, $C_{3-6}$-Alkenyl, $C_{1-6}$-Alkyl, substituiert durch Phenyl oder substituiertes Phenyl, oder $C_{2-6}$-Alkyl, substituiert durch Hydroxy oder $C_{1-6}$-Alkoxy, steht und $R_8$ für Wasserstoff, $C_{1-6}$-Alkyl, $C_{3-6}$-Alkenyl, $C_{1-6}$-Alkyl, substituiert durch Phenyl oder substituiertes Phenyl, Hydroxy-$C_{1-6}$-alkyl, Acyloxy-$C_{1-6}$-alkyl, wobei der Acylteil Benzoyl, substituiertes Benzoyl ist, $C_{1-6}$-Alkanoyl oder $C_{1-6}$-Alkanoyl, substituiert durch Phenyl oder substituiertes Phenyl, $C_{1-6}$-Alkoxy-$C_{1-6}$-alkyl, Aryloxy-$C_{1-6}$-alkyl, wobei die Arylgruppe Phenyl oder substituiertes Phenyl ist, Aryl-$C_{1-6}$-alkyloxy-$C_{1-6}$-alkyl, wobei die Arylgruppe Phenyl oder substituiertes Phenyl ist, Amino-$C_{1-6}$-alkyl, $C_{1-6}$-Alkylamino-$C_{1-6}$-alkyl, Di-$C_{1-6}$-alkylamino-$C_{1-6}$-alkyl, Hydroxy oder $C_{1-6}$-Alkoxy oder die Gruppe $NR_{10}R_{11}$ steht, wobei $R_{10}$ für Wasserstoff, $C_{1-6}$-Alkyl, $C_{1-6}$Alkyl, substituiert durch Hydroxy oder $C_{1-3}$-Alkoxy, $C_{3-6}$-Alkenyl, $C_{1-6}$-Alkyl, substituiert durch Phenyl oder substituiertes Phenyl, oder $C_{1-4}$-Alkyl, substituiert durch einen 5- oder 6-gliedrigen monocyclischen Heteroarylring, enthaltend ein oder mehrere Heteroatome, ausgewählt aus Sauerstoff, Stickstoff und Schwefel, steht, wobei der Heteroarylring unsubstituiert ist oder durch $C_{1-3}$-Alkyl, $C_{1-3}$-Alkoxy, Hydroxy, Amino-$C_{1-6}$-alkyl, $C_{1-6}$-Alkylamino-$C_{1-6}$-alkyl, Di-$C_{1-6}$-alkylamino-$C_{1-6}$-alkyl oder Halogen substituiert ist, und wobei der Heteroarylring mit der Alkylgruppe entweder durch ein Kohlenstoff- oder ein Stickstoffatom verbunden ist, und $R_{11}$ für irgendeine der für $R_{10}$ definierten Gruppen steht oder die Bedeutung $COR_{12}$ haben kann, wobei $R_{12}$ für Wasserstoff, $C_{1-6}$-Alkyl, Phenyl, substituiertes Phenyl, $C_{1-6}$-Alkyl, substituiert durch Phenyl oder substituiertes Phenyl, $C_{1-6}$-Alkoxy, einen 5- oder 6-gliedrigen monocyclischen Heteroarylring, enthaltend ein oder mehrere Heteroatome, ausgewählt aus Sauerstoff, Stickstoff und Schwefel, wobei der Heteroarylring unsubstituiert ist oder durch $C_{1-3}$-Alkyl, $C_{1-3}$-Alkoxy, Hydroxy-

Amino-$C_{1-6}$-alkyl, $C_{1-6}$-Alkylamino-$C_{1-6}$-alkyl, Di-$C_{1-6}$-alkylamino-$C_{1-6}$-alkyl oder Halogen substituiert ist, oder $C_{1-4}$-Alkyl, substituiert durch einen 5- oder 6-gliedrigen monocyclischen Heteroarylring, enthaltend ein oder mehrere Heteroatome, ausgewählt aus Sauerstoff, Stickstoff und Schwefel, wobei der Heteroarylring unsubstituiert ist oder durch $C_{1-3}$-Alkyl, $C_{1-3}$-Alkoxy, Hydroxy, Amino-$C_{1-6}$-alkyl, $C_{1-6}$-Alkylamino-$C_{1-6}$-alkyl, Di-$C_{1-6}$-alkylamino-$C_{1-6}$-alkyl oder Halogen substituiert ist, und wobei der Heteroarylring mit der Alkylgruppe entweder durch ein Kohlenstoff- oder ein Stickstoffatom verbunden ist, steht, oder $R_{11}$ für die Gruppe $SO_2R_{13}$ steht, wobei $R_{13}$ für $C_{1-6}$-Alkyl, Phenyl oder substituiertes Phenyl steht oder $R_{11}$ für die Gruppe

$$\underset{\underset{E}{\|}}{C}NHR_{14}$$

steht, wobei E für Sauerstoff oder Schwefel steht und $R_{14}$ für Wasserstoff, $C_{1-6}$-Alkyl, $C_{3-8}$-Cycloalkyl, Phenyl, substituiertes Phenyl oder $C_{1-6}$-Alkyl, substituiert durch Phenyl oder substituiertes Phenyl, steht, oder $R_{10}$ und $R_{11}$ miteinander genommen die Gruppe $=CR_{15}R_{16}$ bedeuten, wobei $R_{15}$ für Phenyl, substituiertes Phenyl oder einen 5- oder 6-gliedrigen monocyclischen Heteroarylring, enthaltend ein oder mehrere Heteroatome, ausgewählt aus Sauerstoff, Stickstoff und Schwefel, steht, wobei der Heteroarylring unsubstituiert ist oder durch $C_{1-3}$-Alkyl, $C_{1-3}$-Alkoxy, Hydroxy, Amino-$C_{1-6}$-alkyl, $C_{1-6}$-Alkylamino-$C_{1-6}$-alkyl, Di-$C_{1-6}$-alkylamino-$C_{1-6}$-alkyl oder Halogen substituiert ist, und $R_{16}$ für Wasserstoff oder $C_{1-6}$-Alkyl steht, und wobei die Bedeutungen "substituiertes Phenyl" und "substituiertes Benzoyl" Phenyl oder Benzoyl, das durch eine oder mehrere $C_{1-6}$-Alkyl- oder $C_{1-3}$-Alkoxygruppen oder Halogenatome substituiert ist, bedeutet, mit den Maßgaben, daß, wenn $R_2$ für die Gruppe $R_4R_5NAlk$ steht, dann $R_3$ sich in 2-Stellung befindet, und wenn $R_2$ für Wasserstoff steht, dann $R_3$ sich in 3-Stellung befindet.

2. Verbindungen nach Anspruch 1, dadurch gekennzeichnet, daß die Gruppen $R_1$, $R_2$, $R_4$, $R_5$, $R_6$, $R_7$, $R_8$, Y und Alk die folgenden Bedeutungen haben: $R_1$ oder $R_2$ (wenn sie eine andere Bedeutung als die Gruppe $R_4R_5NAlk$ haben): ein Wasserstoffatom, ein Bromatom oder eine $C_{1-3}$-Alkylgruppe oder eine $C_{1-3}$-Alkoxy-$C_{1-3}$-alkylgruppe oder eine Hydroxy-$C_{1-3}$-alkylgruppe, oder wenn $R_1$ oder $R_2$ die Gruppe $R_4R_5Alkyl$ ist, $R_4$: $C_{1-10}$-Alkyl, $C_{5-7}$-Cycloalkyl, $C_{3-6}$-Alkenyl, $C_{1-6}$-Alkyl, substituiert durch Phenyl oder unsubstituiertes Phenyl, $C_{1-4}$-Alkyl, substituiert durch eine Trifluormethylgruppe, Hydroxy-$C_{2-4}$-alkyl, $C_{1-3}$-Alkoxy-$C_{2-4}$-alkyl, Di-$C_{1-3}$-alkylamino-$C_{1-6}$-alkyl oder Heteroaralkyl, wobei der heterocyclische Teil ein Furyl-, Thienyl-, Pyrrolyl-, Pyridinyl-, Pyrimidinyl-, Triazinyl-, Oxazolyl-, Triazolyl- oder Thiazolylring ist und der Alkylenteil eine Methylen-, Ethylen- oder Propylengruppierung ist, und $R_5$: eine Wasserstoff- oder eine Methyl- oder Ethylgruppe, oder $R_4R_5N$ einen 5- bis 8-gliedrigen Ring darstellen können, der gegebenenfalls eine Doppelbindung enthält und/oder durch eine oder zwei $C_{1-3}$-Alkylgruppen oder eine Hydroxygruppe substituiert ist, und/oder ein Sauerstoff- oder Schwefelatom enthält, $R_6$: Wasserstoff, $C_{1-4}$-Alkyl oder $C_{1-3}$-Alkoxy-$C_{2-4}$-alkyl, Y: $CHNO_2$ oder $NR_9$, wobei $R_9$ die Bedeutung Nitro, Cyano, $C_{1-4}$-Alkylsulfonyl, Phenylsulfonyl oder substituiertes Phenylsulfonyl hat, $R_7$: Wasserstoff, $C_{1-4}$-Alkyl oder Hydroxy-$C_{2-4}$-alkyl, $R_8$: Wasserstoff, Hydroxy, $C_{1-4}$-Alkyl, Hydroxy-$C_{1-4}$-alkyl, $C_{1-3}$-Alkoxy-$C_{1-4}$-alkyl, Phenyl-$C_{1-3}$-alkyl, $C_{2-4}$-Alkanoyloxy-$C_{1-4}$-alkyl, Amino-$C_{1-4}$-alkyl, Amino, $C_{1-4}$-Alkylamino oder Di-$C_{1-4}$-alkylamino, Phenyl-$C_{1-3}$-alkylamino, oder eine Heteroaryl-$C_{1-3}$-alkylaminogruppe, wobei der Heteroarylring 5- oder 6-gliedrig ist und ein Heteroatom enthält, oder die Gruppe $NHCOR_{12}$ bedeutet, wobei $R_{12}$ für Wasserstoff, $C_{1-3}$-Alkyl, $C_{1-3}$-Alkoxy, Furyl, Pyridyl, Thiazolyl, Thienyl oder Phenyl, das gegebenenfalls durch eine $C_{1-3}$-Alkyl- oder $C_{1-3}$-Alkoxygruppe substituiert ist, steht, oder die Gruppe $NHSO_2R_{13}$ bedeutet, wobei $R_{13}$ für $C_{1-3}$-Alkyl oder Phenyl, das gegebenenfalls durch eine $C_{1-3}$-Alkyl- oder $C_{1-3}$-Alkoxygruppe substituiert ist, steht, oder für die Gruppe $NHCONHR_{14}$ steht, wobei $R_{14}$ $C_{1-3}$-Alkyl, $C_{5-7}$-Cycloalkyl oder Phenyl, das gegebenenfalls durch eine $C_{1-3}$-Alkyl- oder $C_{1-3}$-Alkoxygruppe substituiert ist, bedeutet, oder die Gruppe $N{=}CHR_{15}$ bedeutet, wobei $R_{15}$ für eine Phenyl- oder Pyridylgruppe steht, Alk die Bedeutung $(CH_2)_p$ hat, wobei p 1 bis 3 ist.

3. Verbindungen nach Anspruch 1 oder 2, dadurch gekennzeichnet, daß sich $R_3$ in 3-Stellung befindet und daß $R_1$ die Gruppe $R_4R_5NAlk$ ist.

4. Verbindungen nach einem der Ansprüche 1 bis 3, dadurch gekennzeichnet, daß Z die Gruppe

$$-\underset{\underset{Y}{\|}}{C}NHR_6$$

bedeutet, wobei Y für die Gruppe $CHNO_2$ steht.

5. Verbindung nach einem der Ansprüche 1 bis 3, dadurch gekennzeichnet, daß Z für die Gruppe

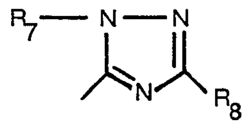

steht, wobei $R_7$ für Wasserstoff oder Methyl steht und $R_8$ für Amino, Hydroxy-$C_{1-4}$-alkyl, $C_{1-3}$-Alkoxy-$C_{1-4}$-alkyl, Benzyl, Formamido, $C_{2-4}$-Alkanoylamino, Amino-$C_{1-4}$-alkyl, $C_{2-4}$-Alkanoyloxy-$C_{1-4}$-alkyl, Hydroxy, Benzoylamino oder Phenylcarbamoylamino steht.

6. Verbindungen nach Anspruch 5, dadurch gekennzeichnet, daß $R_8$ für Amino, Hydroxy-$C_{1-4}$-alkyl, $C_{2-4}$-Alkanoylamino oder Benzyl steht.

7. Verbindungen nach Anspruch 1, dadurch gekennzeichnet, daß sie der Formel (II):

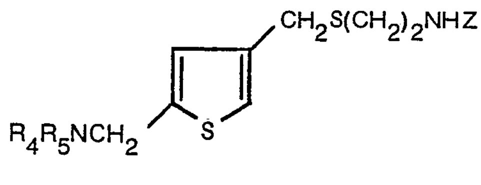

(II)

entsprechen, wobei $R_4$ und $R_5$ Methylgruppen sind oder zusammen mit dem Stickstoffatom, an das die angefügt sind, eine Pyrrolidino-, Piperidino- oder Hexamethyleniminogruppe bilden und Z entweder für

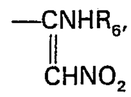

wobei $R_6$ Methyl bedeutet, oder die Gruppe

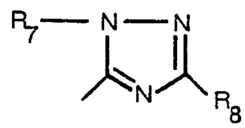

wobei $R_7$ Wasserstoff oder Methyl bedeutet und $R_8$ Amino, Hydroxy-$C_{1-4}$-alkyl, $C_{1-3}$-Alkoxy-$C_{1-4}$-alkyl, Benzyl, Formamido, $C_{2-4}$-Alkanoylamino, Amino-$C_{1-4}$-alkyl, $C_{2-4}$-Alkanoyloxy-$C_{1-4}$-alkyl, Hydroxy, Benzoylamino oder Phenylcarbamoylamino bedeutet, steht.

8. Verbindungen nach Anspruch 1, nämlich
N-Methyl-N'-[2-[[5-(dimethylaminomethyl)-3-thienylmethyl]-thio]-ethyl]-2-nitro-1,1-ethendiamin,
N-Methyl-N'-[2-[[5-(1-pyrrolidinylmethyl)-3-thienylmethyl]-thio]-2-nitro-1,1-ethendiamin,
N-Methyl-N'-[2-[[5-(1-piperidinylmethyl)-3-thienylmethyl]-thio]ethyl]-2-nitro-1,1-ethendiamin,
1-Methyl-5-[[2-[[5-(dimethylaminoethyl)-3-thienylmethyl]-thio]-ethyl]-amino]-1H-1,2,4-triazol-3-methanol,
5-[[2-[[5-(Dimethylaminomethyl)-3-thienyl]-methyl]-thio]-ethyl]-amino-3-phenylmethyl-1H-1,2,4-triazol
und ihre physiologisch annehmbaren Salze.

9. Verbindungen nach Anspruch 1, nämlich
N-[2-[[5-(Dimethylaminomethyl)-4-methyl-2-thienylmethyl]-thio]-ethyl]-N'-methyl-2-nitro-1,1-ethendiamin,
1-Methyl-$N^5$-[2-[[5-[(dimethylamino)-methyl]-4-methyl-2-thienylmethyl]-thio]-ethyl]-1H-1,2,4-triazol-3,5-diamin,
5-[[2-[[[5-(Dimethylaminomethyl)-4-methyl]-2-thienylmethyl]-thio]-ethyl]-amino]-1-methyl-1H-1,2,4-triazol-3-methanol
und ihre physiologisch annehmbaren Salze.

10. Verbindung nach Anspruch 1, nämlich N - [3 - [5 - (Dimethylaminoethyl) - 3 - thienylmethoxy] - propyl] - N' - methyl - 2 - nitro - 1,1 - ethendiamin und seine physiologisch annehmbaren Salze.

11. Verbindungen der Formel (I) gemäß Anspruch 1, dadurch gekennzeichnet, daß einer der Substituenten $R_1$ und $R_2$ für Wasserstoff steht und der andere für $R_4R_5NAlk$ steht und daß $R_3$ für

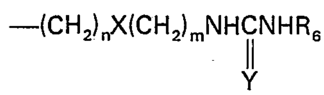

steht, mit der Ausnahme, daß Y nicht die Bedeutung $NR_9$ hat, wo $R_9$ für $C_{1-6}$-Alkylsulfonyl, Phenylsulfonyl oder substituiertes Phenylsulfonyl steht.

12. Verbindungen der Formel (I) nach Anspruch 1, dadurch gekennzeichnet, daß einer der Substituenten $R_1$ und $R_2$ für Wasserstoff steht und daß der andere für $R_4R_5$NAlk steht und daß $R_3$ für

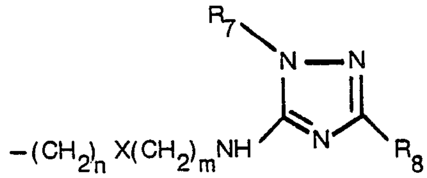

steht, wobei $R_8$ für Wasserstoff, $C_{1-6}$-Alkyl, $C_{3-6}$-Alkenyl, $C_{1-6}$-Alkyl, substituiert durch Phenyl oder substituiertes Phenyl, Hydroxy-$C_{1-6}$-alkyl, $C_{1-6}$-Alkoxy-$C_{1-6}$-alkyl, Hydroxy oder $C_{1-6}$-Alkoxy steht oder $R_8$ für die Gruppe $NR_{10}R_{11}$ steht, wobei $R_{10}$ für Wasserstoff, $C_{1-6}$-Alkyl, Hydroxy-$C_{1-6}$-alkyl, $C_{3-6}$-Alkenyl oder $C_{1-6}$-Alkyl, das durch Phenyl oder substituiertes Phenyl substituiert ist, steht und $R_{11}$ für Wasserstoff, $C_{1-6}$-Alkyl, Hydroxy-$C_{1-6}$-alkyl oder die Gruppe $COR_{12}$ steht, wobei $R_{12}$ für Wasserstoff, $C_{1-6}$-Alkyl, Phenyl, substituiertes Phenyl, $C_{1-6}$-Alkoxy oder $C_{1-6}$-Alkyl, das durch Phenyl oder Substituiertes Phenyl substituiert ist, steht oder $R_{11}$ für die Gruppe $SO_2R_{13}$ steht, wobei $R_{13}$ für $C_{1-6}$-Alkyl, Phenyl oder substituiertes Phenyl steht, oder $R_{10}$ und $R_{11}$ miteinander die Gruppe $=CR_{15}R_{16}$ bilden, wobei $R_{15}$ für Phenyl oder substituiertes Phenyl steht und $R_{16}$ für Wasserstoff oder $C_{1-6}$-Alkyl steht.

13. Verfahren zur Herstellung der Verbindungen der Formel (I) nach Anspruch 1, dadurch gekennzeichnet, daß man

(a) zur Herstellung von Verbindungen, bei denen Z für die Gruppe

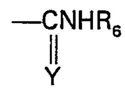

steht, ein Amin der Formel $R_{17}NH_2$ (III) mit einer Verbindung der allgemeinen Formel (IV):

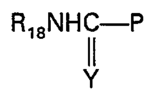

in der eine der Gruppen $R_{17}$ und $R_{18}$ die Gruppe

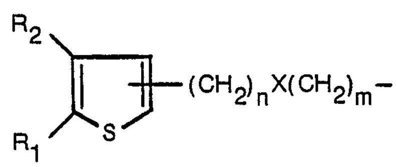

bedeutet und die andere die Gruppe $R_6$ bedeutet, und P eine verlassende Gruppe bedeutet, umsetzt,

(b) zur Herstellung von Verbindungen, bei denen Z die Gruppe

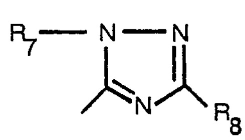

worin $R_8$ eine andere Bedeutung als eine $C_{1-6}$-Alkoxy- oder Acyloxy-$C_{1-6}$-alkylgruppe oder die Gruppe $—N=CR_{15}R_{16}$ hat, eine Verbindung der Formel (VI):

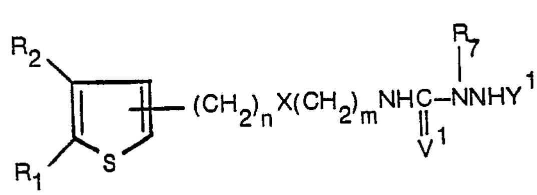

(VI)

worin $V^1$ für

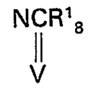

oder NCN steht und $Y^1$ für Wasserstoff steht, wobei V für Sauerstoff oder Schwefel steht, und $R^1{}_8$ eine wie für $R_8$ definierte Gruppe oder eine bei den Bedingungen der Reaktion in eine solche Gruppe umwandelbare Gruppe steht, oder für Halogen oder $C_{1-6}$-Alkoxy steht, oder $V^1$ für NH steht und $Y^1$ für

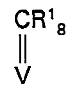

steht, cyclisiert,

(c) zur Herstellung von Verbindungen, bei denen Alk für $CH_2$ steht und Z für die Gruppe

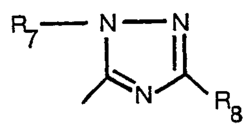

steht, wobei $R_8$ eine andere Bedeutung als die Gruppe —$N{=}CR_{15}R_{16}$ hat, ein Zwischenprodukt, gebildet durch Umsetzung einer Verbindung der Formel (VIII):

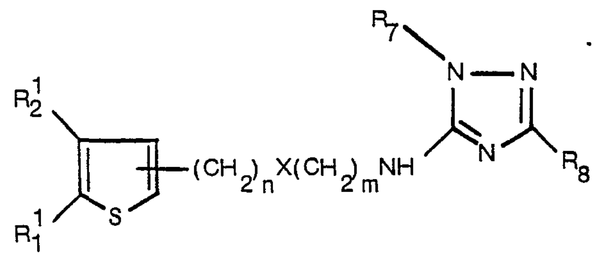

(VIII)

worin einer der Substituenten $R^1{}_1$ und $R^1{}_2$ für Wasserstoff, Halogen oder eine $C_{1-4}$-Alkylgruppe, die gegebenenfalls durch Hydroxy oder $C_{1-4}$-Alkoxy substituiert sein kann, steht und der andere für die Gruppe —CHO steht, mit einem Reagenz $R_4R_5NH$ reduziert,

(d) zur Herstellung von Verbindungen, bei denen $R_8$ eine andere Bedeutung als —$N{=}CR_{15}R_{16}$ hat, eine Verbindung der Formel (I), bei der $R_4$ für Wasserstoff steht, mit einem Aldehyd $R^1{}_4$—CHO unter reduzierenden Bedingungen umsetzt, wobei die Gruppe $R^1{}_4$ eine derartige Bedeutung hat, daß die resultierende Gruppe $R^1{}_4CH_2$ eine Gruppe $R_4$, wie definiert, darstellt,

(e) zur Herstellung von Verbindungen, bei denen Z für die Gruppe

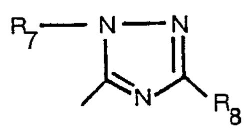

steht, wobei $R_8$ für eine Acyloxy-$C_{1-6}$-alkylgruppe steht, die entsprechende Hydroxy-$C_{1-6}$-alkylverbindung mit einer geeigneten Säure bei erehöhter Temperatur behandelt,

(f) zur Herstellung von Verbindungen, bei denen Z für die Gruppe

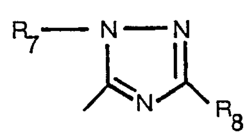

steht, wobei die Gruppe $R_8$ für die Gruppe —$N{=}CR_{15}R_{16}$ steht, eine Verbindung der Formel (I), bei der $R_8$ eine $NH_2$-Gruppe ist, mit einem Aldehyd oder Keton $R_{15}R_{16}CO$ umsetzt,

(g) zur Herstellung von Verbindungen, bei denen Z für die Gruppe

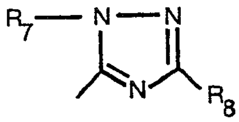

steht, wobei $R_8$ für die Gruppe $NR_{10}R_{11}$ steht, wobei $R_{11}$ für $—COR_{12}$, $—SO_2R_{13}$ oder $—C(=E)NHR_{14}$ steht, eine Verbindung der Formel (I), wobei $R_8$ für die Gruppe $—NHR_{10}$ steht und $R_1$, $R_2$, $R_7$ und $R_{10}$ die vorstehend im Zusammenhang mit der Formel (I) angegebenen Definitionen besitzen oder Gruppen darstellen, die ohne weiteres in solche Gruppen umwandelbar sind, mit einem Reagenz behandelt, das dazu imstande ist, das Wasserstoffatom in der Gruppe $NHR_{10}$ durch die Gruppe $R_{11}$ auszutauschen,

(h) eine Verbindung der Formel (I), bei der die Gruppe $R_4R_5N$ durch eine quaternäre Ammoniumgruppe ersetzt worden ist, mit einem Amin $R_4R_5NH$ behandelt,

und wenn die Verbindung der Formel (I) in Form einer freien Base hergestellt worden ist, gegebenenfalls die freie Base in ein Salz umwandelt.

14. Arzneimittel, dadurch gekennzeichnet, daß es eine Verbindung nach einem der Ansprüche 1 bis 12 zusammen mit mindestens einem pharmazeutisch annehmbaren Träger oder Verdünnungsmittel und gegebenenfalls einem oder mehreren weiteren Wirkstoffen enthält.